(19) Europäisches Patentamt
European Patent Office
Office européen des brevets

(11) Publication number: 0 264 118 B1

(12) # EUROPEAN PATENT SPECIFICATION

(45) Date of publication of patent specification: 04.08.93

(51) Int. Cl.5: C12N 15/62, C12P 21/02, //(C12P21/02,C12R1:19, C12N9:12)

(21) Application number: 87115016.5

(22) Date of filing: 14.10.87

(54) **Novel method for preparing proteins.**

(30) Priority: 14.10.86 JP 245048/86
23.12.86 JP 314603/86

(43) Date of publication of application:
20.04.88 Bulletin 88/16

(45) Publication of the grant of the patent:
04.08.93 Bulletin 93/31

(84) Designated Contracting States:
AT BE CH DE ES FR GB GR IT LI LU NL SE

(56) References cited:
EP-A- 0 094 506
WO-A-85/03522

THE EMBO JOURNAL, vol. 3, no. 13, May 1984, pages 3317-3322, IRL Press, Oxford, GB; B. REISS et al.: "Protein fusions with the kanamycin resistance gene from transposen Tn5"

(73) Proprietor: SHIONOGI SEIYAKU KABUSHIKI KAISHA trading under the name of SHIONOGI & CO. LTD.
12, 3-chome, Dosho-machi Higashi-ku
Osaka(JP)

(72) Inventor: Teraoka, Hiroshi
2-47-10, Takakuradai
Sakai-shi Osaka(JP)
Inventor: Shin, Masaru
3-110, Koryo-cho, Kita-ku
Kobe-shi Hyogo(JP)
Inventor: Ohara, Osamu
4-21-15-303, Kamishinden
Toyonaka-shi Osaka(JP)
Inventor: Kikuchi, Norihisa
1-1-8-705, Takasu-cho
Nishinomiya-shi Hyogo(JP)

(74) Representative: Vossius & Partner
Siebertstrasse 4 P.O. Box 86 07 67
W-8000 München 86 (DE)

EP 0 264 118 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**GENE, vol. 40, 1985, pages 191-201, Elsevier Science Publishers; M.E. BRAWNER et al.: "Characterization of Streptomyces promoter sequences using the Escherichia coli galactokinase gene"**

**SCIENCE, vol. 198, 9th December 1977, pages 1056-1063; K. ITAKURA et al.: "Expression in Escherichia coli of a chemically synthesized gene for the hormone somatostatin"**

**NATURE, vol. 298, 26th August 1982, pages 857-859, Macmillan Journals Ltd; R. ZAKUT et al.: "Nucleotide sequence of the rat skeletal muscle actin gene"**

**JOURNAL OF BIOCHEMISTRY, vol. 98, no. 3, 1985, pages 687-694; N. KIKUCHI et al.: "The multiplicity of human pancreatic secretory trypsin inhibitor"**

**THE JOURNAL OF BIOCHEMISTRY, vol. 102, no. 3, September 1987, pages 607-612, Tokyo, JP; N. KIKUCHI et al.: "Production of recombinant human pancreatic secretory trypsin inhibitor by Escherichia coli"**

## Description

This invention relates to a method for preparing protein by transforming a microorganism with a vector capable of expressing a desired protein as a fused protein with the N-terminal portion of the APH, making said microorganism producing said fused protein and obtaining said desired protein.

Along with the recent remarkable progress in genetic engineering, various useful peptides are being produced in microorganisms. Among others, peptides with relatively short chains such as human insulin and human somatostatin are, when produced in microorganisms, easily decomposed by protease and the production efficiency is low. Therefore, it has been attempted to produce a desired protein in microorganisms as a fused protein (for example, as disclosed in the Japanese Unexamed Patent Publication No. 54-92696). Where the product is lethal to the host or inhibits its proliferation and the production efficiency is low, it is possible to raise the production efficiency by making the product as a fused protein which is inactive to the host.

Transposon Tn5 contains a gene coding for APH (aminoglycoside 3'-phosphotransferase II) which provides a microorganism with neomycin- or kanamycin- resistance. The base sequence of this gene in transposon Tn5 has already been known (Gene 19, 327, 1982) and a plasmid containing this base sequence is commercially available (for example, pNEO by Pharmacia). This base sequence and the amino acid sequence of APH derived therefrom are shown in Fig. 1.

Trypsin inhibitors derived from the pancreas are classified into two types, that is pancreatic secretory trypsin inhibitor (PSTI) and basic pancreatic trypsin inhibitor. The former is present in mammals and is, besides the pancreas, distributed in kidney, lung, spleen, liver and brain. The latter exists in the pancreas and other organs of ruminants, but is not found in man and other mammals. Pubols et al. (J. Biol. Chem. 249, 2235, 1974) and Feinstein et al. (Eur. J. Biochem. 43, 569, 1974), respectively, isolated and purified human PSTI from human pancreatic juice. Its structure was elucidated by Greene et al. (Methods Enzymol. 45, 813, 1976). However,the amino acid sequence of PSTI reported by Greene et al. is different from the amino acid sequence shown in Fig. 2 in the 21st and 29th position (J. Biochem. 98, 687, 1985). The human PSTI is, as shown in Fig. 2,a polypeptide composed of 56 amino acid residues. Its molecular weight is 6242. The SH group of Cys forms a disulfide bond between the 9th and 38th position, 16th and 35th position and 24th and 56th position of the amino acid sequence. A free SH group is not present. Eddeland et al. (Hoppe-Seyler's Z. Physiol. Chem. 359, 671, 1978) and Kitahara et al. (Biomed. J. 3, 1119, 1979) established the radioimmunoassay system for measuring PSTI in blood using human PSTI obtained from pancreatic juice as antigen. Yamamoto et al. (Biochem. Biophys. Res. Commun. 132, 605, 1985) determined the DNA sequence of human PSTI (see Fig. 2).

Autolysis of acute pancreatitis is induced by proteolytic enzymes. It is said because a small quantity of trypsin activated by some cause activates trypsinogen and other zymogene in a chain reaction. The PSTI existing in the pancreatic acinar cell is secreted into the pancreatic juice together with pancreatic enzymes and inhibits the activation of trypsin occurring in the pancreatic duct. Furthermore, a part of this PSTI moves into the blood as a released inhibitor and exists in a free state (Cholecyst and Pancreas, 2, 231, 1981). The blood level of PSTI fluctuates notably in pancreatic diseases, particular in acute pancreatic diseases, and the period with the high level is longer than that of amylase. Also, indifferent to the protease inhibitor, fluctuations of the blood level of PSTI sensibly express the pancreatic stress (Cholecyst and Pancreas, 3, 383 , 1982). Therefore, by measuring the blood level of PSTI, the diagnosis and process observation of pancreatic diseases may be enabled.

Rat actin is a protein which forms the skeletal muscles of rats.

The gene encoding this actin has been already cloned (Biophysics, vol. 25, Supplement, pp S78, 1985) (see Fig. 5).

Human PSTI and rat actin have not yet been produced by utilizing genetic engineering, but expression of human insulin or human sonatostatin as a fused protein with β-galactosidase has already been known as a method which may be applied to express a peptide with a relatively short chain such as human PSTI in microorganisms(see above). This system, however, cannot be applied to all peptides. Therefore, it is very important to find other systems for expressing a desired peptide as a fused protein in microorganisms.

Genes coding for APH are commercially available. They are for instance contained in transposon Tn5. However, no attempt has been made yet to express a desired peptide in a microorganism as a fused protein with APH.

For diagnosis and process observation of pancreatic diseases measurement of PSTI by RIA is widely employed. For this measurement human PSTI is indispensable as a standard reagent. Since it is until now isolated and purified from human pancreatic juice, the available quantity is limited. It was so far impossible to obtain it in large quantities.

DNA encoding rat actin has been already cloned as mentioned above. However, it is shown by the experiments of the inventors (see Examples), that rat actin is hardly produced if it is expressed in E. coli as a non-fused protein.

The technical problem underlying the present invention is to provide a method for preparing a desired protein by transforming a microorganism with a vector capable of expressing said protein as a fused protein with the N-terminal portion of APH, cultivating said microorganism to produce said fused protein and recovering said desired protein.

According to the present invention it was found that a large quantity of APH is produced in microorganisms when the APH gene is placed downstream of a suitable promoter. It was also found that, by making use of the high expression capability of APH, a desired protein can be produced in microorganisms as a fused protein.

This invention, therefore, discloses a method for preparing a desired protein, such as human PSTI and rat actin, by firstly connecting a gene encoding it to an shortened APH gene. The obtained fused gene is then introduced into a vector, where it is placed downstream of a proper promoter. This recombinant vector is tranferred into a host which produces the encoded protein. Finally, the desired protein is recovered from said fused protein. This method is useful for the production of various types of proteins.

This invention relates to a method for the production of a desired protein which makes use of the high expression ability of APH gene, that is, the excellent efficiency of transcription to mRNA and translation from mRNA into a polypeptide. If the desired protein is a relatively short chain it is produced according to the present invention as a fused protein with the long-chain APH in the microorganism. Thus the decomposition of the desired protein by proteases in the microorganism can be avoided. It is also possible to deprive the harmful activity of the desired protein by the fusion. The excellent production efficiency of this invention is considered to be mainly due to the improvement of efficiency of translation from mRNA into peptide. It seems particularly effective for the expression of a peptide which had a low expression efficiency due to the low translation efficiency.

Fig. 1 shows a DNA sequence of the APH gene and the amino acid sequence derived from this DNA sequence. Fig. 2 shows the DNA and the amino acid sequence of human PSTI. Fig. 3 shows the DNA sequence of the synthetic human PSTI gene used in an example, the corresponding amino acid sequence and the synthesizing strategy of the DNA sequence. Fig. 4 shows a restriction map of the APH gene and its flanking regions. Fig. 5 is an outline of the construction of pUCKMFPSTI. Fig. 6 is an outline of the construction of pUCKMF. Fig. 7 is a DNA sequence of a rat skeletal muscle actin gene and the derived amino acid sequence. Fig. 8 is an outline of the construction of pUCACT. Fig. 9 is an outline of the construction of pUCKMFACT. Fig. 10 is an outline of the construction of pUCKMF1. Fig. 11 is an outline of the construction of pUCKMFACT1. Fig. 12 is an outline of the construction of pUCKMACT. Fig. 13 shows the DNA sequence near the synthetic oligonucleotide sequence inserted in constructing pUCKMACT. Fig. 14 is an outline drawing of a fused protein of APH and rat skeletal muscle actin as expressed in the examples. Fig. 15 shows the relation between length and production output of a peptide fused with rat skeletal muscle actin.

According to the present invention it was discovered that a large quantitiy of APH is produced in microorganisms when the APH gene is placed downstream of a proper promoter. It was also found that, by making use of the high expression capability of APH, human PSTI and rat actin can be produced in microorganisms as fused proteins.

This invention, therefore, discloses a method for preparing a desired protein, such as human PSTI and rat actin, by firstly connecting a gene encoding it to a shortened APH gene. The obtained fused gene is then introduced into a vector, where it is placed downstream of a proper promoter. This recombinant vector is tranferred into a host which produces the encoded protein. Finally, the desired protein is recovered from said fused protein. This method is not only useful for the production of said human PSTI and rat actin but also for the production of other proteins.

Incidentally, the APH gene may contain not only the structural gene to code APH but also its promoter and/or ribosome-binding sequence. The fused gene comprising the APH gene containing the APH promoter and a gene encoding a desired protein may be placed under the control of a strong promoter such as the lac promoter, Trp promoter or Tac promoter. In another embodiment the fused gene comprising the ribosome-binding sequence and the structural gene of APH, but not the APH promoter, together with a gene coding for desired protein may be placed under the control of said promoters. It is also considered that the vicinity of the ribosome-binding sequence of said APH gene may be replaced by other suitable ribosome-binding sequences. Besides, it is not always necessary that the APH gene contains the complete APH structural gene. It is sufficient if it comprises a DNA sequence encoding 13 or more amino acid residues of the N-terminus, preferably 24 or more amino acid residues. That is, the potent expression efficiency of this invention is considered to be caused mainly by the high translation efficiency from mRNA to protein. What is considered to be important is that the sequence comprises the ribosome-binding sequence of the APH gene and a sequence encoding stretches of amino acid residues from the N-terminus of APH, which is responsible for initiation of translation. For example, when a synthetic human PSTI DNA is connected to the HindIII-TaqI restriction fragment of pNEO (Pharmacia) (containing the APH promoter and the gene coding for the amino acid residues of the N-terminus of APH up to the 82nd position, corresponding to the DNA sequence from positions -350 to 246 in Fig. 1) and placed under the control of the lac promoter of pUC13 (Takara Shuzo Co., Ltd.), a vector is constructed which expresses a fused protein of APH and human PSTI. Since not only the sequence shown in Fig. 1 but also sequences in which some bases of the APH gene are replaced, eliminated or inserted have high translation efficiency, the use of such variant APH genes in the method of the present invention should be considered to be included in the scope of this invention. In this case, naturally, it is supposed that the amino acid sequences of the APH will be greatly altered, but as far as the DNA has similar efficiency, the method in which such variant APH is used is also encompassed by the scope of this invention.

Examples of the vectors carrying the fused gene encoding APH and a desired protein are: pUC13, p$\beta$-gall3C, pOP203-13, pUC9, pUC8, pEA300, ptrpL1, pBN70, pWT111, pWT121, pWT131, pKK223-3, pDR540, pDR720, pYEJ001, pPL-lambda, pKC30, pKC31, pAS1, pLC24, pHUB4, pIN-I, pIN-II, pIN-III, pC194, pC221, pUB112, pT127, pSA0503 and pE194. But these are not limitative and any vector capable of carrying said fused gene and expressing it in a microorganism may be used. In addition, all vectors generally used for transformation in this field may be used. By properly selecting these vectors depending on the host and placing said fused gene under the control of a suitable promoter, the fused protein of APH and a desired protein can be expressed.

When E. coli, for example, is used as the host, a desired protein can be produced efficiently.

The desired protein can be easily recovered from said fused protein, using cyanogen bromide if a suitable amino acid binding sequence such as Met or sequence containing Met, is contained in the fused protein between APH and the desired protein. Other methods are also applicable. For example, the method of inserting Cys between two proteins and cutting with 2-nitro-5-thiocyanobenzoic acid, the method of inserting Asn-Gly and cutting with hydroxylamine, the method of inserting Trp and cutting with 2-(2-nitrophenylsulphenyl)-3-methyl-3-bromo indole, the method of inserting Lys or Arg and cutting with trypsin and the method of inserting -Ile-Glu-Gly-Arg- and cutting with blood coagulation factor Xa. These methods, which are generally employed, may be properly selected in consideration of the amino acid sequence of the intended protein.

Besides, not only for the purpose of these cuttings, a few binding base sequences may inevitably be inserted owing to the construction of vectors, but influence on the production efficiency of an intended protein are insignificant.

A desired protein cut off from the fused protein may be routinely purified by properly combining chromatography, affinity chromatography, centrifugation and other operations.

The gene encoding human PSTI has already been cloned by Yamamoto et al. (see above) from human pancreatic cells and its sequence has already been determined. Since it is a relatively short chain, it is advantageous to use a synthetic human PSTI gene. Of course, it may be also prepared according to the usual method from human pancreatic cells as attempted by Yamamoto et al. (see above). This sequence may be any of the sequences coding for the amino acid sequence of human PSTI shown in Fig. 2 as well as natural DNA sequences as shown in Fig. 2. When connecting the human PSTI gene with an APH gene it is preferred to insert between the APH gene and the 5′-terminus of the human PSTI gene the sequence ATG which encodes a Met residue. In this case the human PSTI can easily be recovered from the expressed fused protein by treating it with cyanogen bromide.

When rat actin is expressed in a microorganism as mature non-fusion actin, it is hardly produced. However, if it is fused with more than 13 residues of the amino terminus of APH, it is produced efficiently in the microorganism. Other proteins can also be produced in high yields if they are fused with APH polypeptides of similar length.

The method of the present invention is explained in more detail in the following examples. These examples relating to human PSTI and rat actin, however, are not construed to limit the scope of the present invention.

Example 1: Manufacture of human PSTI

The base sequence of the human PSTI gene was prepared on the basis of the sequence determined by Yamamoto et al. (Biochem. Biophys. Res. Commun., 132, 605, 1985) and a sequence ATG encoding Met was bonded immediately before the structural gene of this mature protein and the termination codon TGA immediately after. Furthermore, a certain base sequence was added thereto so as to possess the recognition site of restriction enzyme AccI at the 5' terminal of this sequence and the recognition site of restriction enzyme BamHI at the 3' terminal, thus designing to form a double strand comprising two chains with chain length of 179 and 181.

Twenty types of short-chain DNA fragments were chemically synthesized in two groups, one of which forms the DNA chain containing the base sequence coding for the amino acid sequence of PSTI and another of which forms a DNA chain complementary to the coding chain, when bonded in a proper sequence order. These fragments, when all types were mixed together, form a double strand structure possessing sticky ends as the recognition sites of the restriction enzymes by forming hydrogen bond in the mutually complementary portion (Fig. 3).

A total of 20 types of fragments of U-1 to U-10 and L-1 to L-10 shown in Fig. 3 were prepared by using a nucleic acid automatic synthesizer GENET A-II (Nippon Zeon). Each of the obtained fragments was refined by the gel chromatography using Sephadex G-50 and the high performance liquid chromatography using a reverse phase silica gel column (Nucleosil 10C18, 10 x 250 mm).

Since the synthesized oligonucleotide does not possess a phosphoric acid group at the 5' terminal, it cannot be directly used in the ligation reaction by T4 DNA ligase. Of these 20 synthetic oligonucleotides, 18 types except U-1 and L-10 were applied in the enzymatic addition reaction of a phosphoric acid group to the 5' terminal. The phosphoric acid group addition reaction is conducted by using T4 polynucleotide kinase (Takara Shuzo Co., Ltd.). About 300 pmol of each oligonucleotide is dissolved in 25 $\mu$l of kinase reaction solution (50mM tris-hydrochloride buffer, 10mM magnesium chloride, 10 mM 2-mercaptoethanol, about 1000 pmol ATP, pH 7.6) and 3 units of the enzyme are added to the reaction solution to start the reaction. The reaction is performed for 1 hour at 37°C. By heat treatment (65°C, 20 minutes), the T4 polynucleotide kinase is devitalized and the mixture is directly used in the following ligation reaction. By mixing 50 pmol each of the 18 synthetic oligonucleotides U-2 to U-10 and L-1 to L-9 phosphorylated and 2 synthetic oligouncleotides U-1 and L-10 without phosphorylation, a ligation reaction solution is prepared. The ligation solution is first heated to 80°C for 2 minutes and gradually cooled to 20°C. Then, adding dithiothreitol, ATP, and T4 DNA ligase, the ligation reaction is performed. The final ligation reaction solution (200 $\mu$l) contains 66 mM tris-hydrochloride buffer, 6.6 mM magnesium chloride, 10 mM dithiothreitol, 1 mM ATP and 700 units T4 DNA ligase (Takara Shuzo Co., Ltd.), and this reaction is continued for 5 days at 4°C. These are basically conforming to the method mentioned in Nucleic Acids Res. 13, 2959 (1985). After the ligation reaction, phenol extraction and ethanol precipition are conducted according to the conventional methods and the polyacrylamide gel electrophoresis is conducted in tris-borate buffer solution for separation. The desired DNA fragments of ca. 180 bp are recovered electrophoretically by using DEAE membrane. The DNA separated on the gel is stained with ethidium bromide and after inserting the DEAE-membrane (Schleicher & Schuell) into the gel near the desired DNA band, the DNA band is adsorbed on the DEAE-membrane electrophoretically. After the termination of migration of the DNA band, the DNA is eluted from the DEAE-membrane with 1.0 M sodium chloride-10 mM tris-hydrochloride buffer (pH 8.0)-1 mM EDTA and the DNA is recovered by ethanol sedimentation. (This method is general and described in details, for example, in "Molecular Cloning," Cold Spring Harbor Laboratory, New York, 1982). These recovered DNA fragments are inserted into M13 phage vector in order to determine the base sequence. M13 mp10 vector (Takara Shuzo Co., Ltd.) is digested by restriction enzymes AccI and BamHI. This linearized mp10 vector and the recovered DNA fragments are linked together by using T4 DNA ligase. The ligation reaction is performed nearly in the the same condition as that of the oligonucleotides described above except that the reaction temperature and time are 12°C and 16 hours. DNA after the ligation reaction is used in transformation according to the method mentioned in Molecular Cloning (Cold Spring Harbor Laboratory, New York, 250-251, 1982). The transformation was conducted by mixing competent cells obtained by treating E. coli K-12 strain JM103 culture in the logarithmic growth phase with calcium chloride at 0°C with the DNA after the ligation reaction, incubating it in ice and treating it at 42°C for 2 minutes. Since the E. coli infected by M13 phages are retarded in the growth rate, they appear as plaque. The JM103 having the DNA is added to 20 $\mu$l of 100 mM isopropyl-$\beta$-D-thiogalactoside,50 $\mu$l of 2% 5-bromo-4-chloro-3-indoryl-$\beta$-galactoside, 0.2 ml of JM103 culture in logarithmic growth phase and 3 ml of soft agar solution (0.6% agar solution), which are applied on a solidified 1.5% agar plate. The used agar contains TY medium (8 g of trypton, 5 g of yeast extract and 5 g of sodium chloride in 1 liter of water). After incubating overnight at 37°C, the transformant forms a plaque. A transformant by M13mp10 itself forms a blue plaque, but a transformation by the M13 phage with deletion and insertion of the DNA fragment leads to the formation of a colorless plaque. According to the Messing's method (Methods Enzymol. 101, 20-28, 1983), a single-stranded phage DNA is prepared in the following manner. Overnight culture (1 ml) of E. coli. K-12 strain JM103 is transplanted into 100 ml of 2 x TY medium (2 x TY medium: 16 g bactotrypton, 10 g yeast extract, 5 g sodium chloride/liter) and incubated for 2 hours at 37°C under shaking. This culture is divided into 5 ml aliquots. The agar on which a plaque was formed is cut out and inoculated into the above aliquot using a capillary pipet. Afterwards, by further incubating the solution for 5 hours at 37°C, infection of M13 phage and its release into the medium are effected. The organisms in this culture are used in preparation of replicative double-stranded DNA and the medium supernatant from which the organisms are removed is used in preparation of single-stranded phage DNA. To the medium supernatant (4 ml), 800 µl of a solution of 20% polyethylene glycol and 2.5 M sodium chloride is added and the phages are harvested by centrifugation. The phages are dissolved in 500 µl of 10 mM tris-hydrochloride buffer and 1 mM EDTA (pH 8.0) and single-stranded phage DNA is collected by phenol extraction and ethanol sedimentation. Preparation of replicative double-stranded DNA from phage-infected organisms conforms to the conventional sodium hydroxide-sodium dodecyl sulfate (SDS) method (Nucleic Acids Res. 7, 1513-1523, 1979). The organisms obtained from 5 ml of the culture are suspended in 100 µl of 50 mM glucose, 25 mM tris-hydrochloride (pH 8.0), 10 mM EDTA and 1 mg/ml lysozyme and let stand at room temperature for 5 minutes. To the resultant 200 µl of 0.2 M sodium hydroxide-1% SDS is added, mixed gently and let stand in ice for 5 minutes. Then, adding and mixing 150 µl of 5 M potassium acetate (pH 5.2), the mixture is allowed to stand in ice for 5 minutes or more. After centrifugation, two volumes of ethanol are added to the supernatant and the sediment is recovered. After dissolving the sediment, ethanol sedimentation is effected again . By this method, a replicative double-stranded DNA is prepared from the colorless plaque. It is subjected to double digestion with Acc I and BamHI and it is confirmed that DNA fragments of about 180 bp are produced. Then, the single-stranded phage DNA prepared from the same plaque is used in the determination of DNA base sequence by the dideoxy method (Science 214, 1205-1210, 1981). The base sequence is determined by using M13 sequencing kit (Takara Shuzo Co., Ltd.). The clone obtained in this way was confirmed to contain the whole range of the structural gene of the PSTI. The replicative double-stranded DNA in which the base sequence has been confirmed is utilized in the next construction of an expression plasmid.

The expression plasmid of PSTI is constructed by the ligation of three fragments shown below.

a) AccI-BamHI fragment of about 180 bp obtained in the above ligation of synthetic oligonucleotides of which base sequence has been confirmed.

b) DNA fragment of about 2.8 Kbp obtained by HindIII-BamHI digestion of pUC13 (Takara Shuzo Co., Ltd.).

c) DNA fragment of about 600 bp obtained by digesting pNEO (containing APH gene of Tn5, Pharmacia) with HindIII and further digesting with TaqI (corresponding to the DNA sequence in positions -350 to 246 in Fig. 2; see Fig. 4).

Of these fragments, fragments a and c were isolated by polyacrylamide gel electrophoresis, recovered by using DEAE membranes and used in the ligation. Fragment b was, after confirmation of double digestion, extracted with phenol, sedimented by ethanol and used for the ligation. The expression plasmid obtained by the ligation of these three fragments expresses a fused protein with the PSTI fused downstream of the amino terminal 82 residues of APH encoded in transposon Tn5. The ligation of these three fragments is also performed by using T4 DNA ligase in the same manner as stated above. The DNA ligated is used in transformation according to the method mentioned in Molecular Cloning (op. cit.). E. coli K-12 strain C600 or AG-1 is used as DNA recipient. Since the transformant acquires resistance to ampicillin, it forms a colony on an agar plate (LB medium; 10 g trypton, 5 g yeast extract and 5 g sodium chloride in one liter) containing ampicillin. Of the emerging colonies, 12 are transplanted on 5 ml of LB medium containing 40 µg/ml of ampicillin by means of platinum loop and incubated for 16 hours at 37°C. The organisms are then harvested by centrifugation and the plasmid is analyzed by the sodium hydroxide-SDS method as mentioned above. The intended plasmid contains a unique restriction site with respect to Hind III, BamHI and PstI. Hence, it is the intended plasmid pUCKMFPSTI that a DNA band of about 3.6 Kb in linear form is produced by digestion with each of the restriction enzymes (see Fig. 5). The clone possessing the pUCKMFPSTI is used in the following expression.

The clone confirmed to carry the intended plasmid is incubated in LB medium (containing 40 µg/ml ampicillin) and stored at -70°C in the presence of 50% glycerin. This bacterial preservation solution (10 µl) is added to 5 ml of LB medium containing ampicillin and incubated for 8 hours at 37°C. This culture (100 µl) is added to 5 ml of M9 medium containing ampicillin (M9 medium: 6 g of disodium hydrogen-phosphate, 3 g of sodium dihydrogen-phosphate, 0.5 g of sodium chloride and 1 g of ammonium chloride are dissolved in 1 liter and sterilized, and then magnesium sulfate and calcium chloride are added until the final concentration becomes 2 mM and 0.1 mM, respectively, and further supplemented 40 µg/ml of ampicillin, 0.5% of glucose and 0.5% of casamino acid) and incubated for 24 hours at 37°C. After incubation, the organisms are harvested by centrifugation and used in the following analysis.

A small portion of the organisms is used as a sample for the analysis in polyacrylamide gel electrophoresis in the presence of SDS (SDS-PAGE). The bacterial protein is solubilized in 0.1 M tris-hydrochloride buffer (pH 6.8), 1% SDS, 1% 2-mercaptoethanol and 20% glycerol, extracted and analyzed in gel electrophoresis. In this state, the fused protein containing PSTI appears as one of the principal bands at a position corresponding to an expected molecular weight of 15000 and it is revealed to be expressed in the E. coli. When the E. coli was sonicated, separated into soluble protein fraction and insoluble protein fraction by centrifugation and investigated by SDS-PAGE, the fused protein was found to be mainly present in the insoluble protein fraction.

The above microorganisms (6 g) were suspended in 20 ml of 0.1 M tris-hydrochloride (pH 7.0) containing 5mM EDTA and centrifuged at 12000 g for 10 minutes. After repeating the same operation, the resultant was suspended in 15 ml of 0.1 M tris-hydrochloride (pH 7.0) containing 5 mM EDTA, 50 mM benzamidine and 1 mM phenylmethylsulfonyl fluoride and crushed three times by using a French press at a pressure of 400 kg/cm$^2$. Pellet (1.05 g) obtained by centrifugation at 23000 g for 20 minutes was dissolved in 10 ml of 0.1 M sodium phosphate (pH 7.0) containing 20 mM dithiothreitol (DTT) and a protein denaturing reagent and subjected to gel filtration on a column of Sephacryl S-200 (2.6 x 79 cm), which had been equilibrated with 0.1 M tris-hydrochloride (pH 7.2) containing 1 mM DTT and 7 M urea. Fractions of molecular weight of about 17000 were collected, dialyzed against distilled water and then lyophilized. The lyophilized material was dissolved in 2 ml of 70% formic acid and then reacted with 160 mg of cyanogen bromide for 6 hours at room temperature. The reaction was stopped by adding 18 ml of distilled water followed by lyophilization. The lyophilized product was dissolved in 2 ml of 0.5 M tris-hydrochloride (pH 8.1) containing 2 mM EDTA and 6 M guanidine hydrochloride and 100 µl of 2-mercaptoethanol was added. The mixture was allowed to react at 37 °C for 4 hours under nitrogen atmosphere and the product was dialyzed against distilled water. The dialyzed material was centrifuged at 10000 g for 1 minute, and to 6 ml of supernatant 172 mg of NaCl and 320 µl of 1 M tris-hydrochloride (pH 8.0) were added and the mixture was applied to an bovine trypsin-CH-Sepharose 4B column previously equilibrated with 0.05 M Tris-HCl (pH 8.0) containing 0.5 M NaCl. The column was washed with the same buffer and then distilled water. PSTI fractions eluted with 10 mM hydrochloric acid were combined and then lyophilized to obtain 1.55 mg of the refined product.

Recombinant human PSTI (rHn-PSTI) (12 µg) was hydrolysed in sealed, evacuated test tube (10 x 90 mm) with 50 µl of 4 M methanesulfonic acid (containing 0.2% 3-(2- aminoethyl)indole) for 24 hours at 110 °C. Amino acid analysis was performed with a Hitachi amino acid analyzer (model 835). The amino acid composition of rHn-PSTI is as shown in Table 1, which perfectly coincided with that of natural human PSTI.

Table 1

| Amino acid | Experimental value | Theoretical value |
|---|---|---|
| Aspartic acid | 7.8 | 8 |
| Threonine | 3.8 | 4 |
| Serine | 2.8 | 3 |
| Glutamic acid | 6.2 | 6 |
| Proline | 2.9 | 3 |
| Glycine | 5.2 | 5 |
| Alanine | 1.4 | 1 |
| Cystine | 2.5 | 3 |
| Valine | 2.0 | 2 |
| Methionine | 0.0 | 0 |
| Isoleucine | 2.8 | 3 |
| Leucine | 4.0 | 4 |
| Tyrosine | 2.9 | 3 |
| Phenylalanine | 1.2 | 1 |
| Lysine | 3.8 | 4 |
| Histidine | 0.0 | 0 |
| Tryptophan | 0.0 | 0 |
| Arginine | 3.0 | 3 |

Partial amino-terminal amino acid sequence of rHn-PSTI was analysed by Edman's method (modified method by Iwanaga et al., Eur. J. Biochem. 8. 189-199, 1969), to be Asp-Ser-Leu, which agreed with that of natural human PSTI. Furthermore, rHu-PSTI inhibited bovine trypsin activiy stoichiometrically and as for the immuno reactivity against anti-natural human PSTI (rabbit antiserum polyclonal), its dilution curve agreed with that of natural human PSTI.

Example 2: Manufacture of rat actin

In order to construct the expression plasmid for fused protein, several vectors are preliminarily constructed. First of all, a HindIII-SalI fragment of transposon Tn5 (derived from pNEO, Pharmacia) is inserted into the HindIII-SalI site of pUC13 (Takara Shuzo Co., Ltd.) (see Fig. 6). Since this DNA perfectly contains the APH gene of transposon Tn5, the E. coli in which the intended DNA is introduced acquires resistance to kanamycin and by making use of this nature a transformed cell of E. coli can be selected. pUC13 is subjected to double digestion with restriction enzymes HindIII and SalI. Conditions of each enzyme digestion conform to the instruction of the supplier of the enzyme. Similarly, pNEO is subjected to double digestion with HindIII and SalI and the obtained DNA fragment of about 1.5 Kb is separated by agarose gel electrophoresis and recovered by using DEAE membrane (Schleicher & Schuell). The DNA in the gel is fluorescently stained by ethidium bromide and then the DEAE membrane is inserted near the DNA band, which is electrophoretically moved to the DEAE membrane. The DNA adsorbed on the DEAE membrane is eluted at 65°C with 1 M sodium chloride-10 mM tris-hydrochloride buffer (pH 8.0)-1 mM ethylenediaminetetraacetic acid (EDTA) and desalted and concentrated by ethanol sedimentation. This procedure is conventional, as described in the literature (Maniatis, T., Fritsch, E. F. & Sambrook, J. "Molecular Cloning," Cold Spring Harbor Laboratory, 1982). For ligation of two fragments, T4 DNA ligase (Takara Shuzo Co., Ltd.) in used and the reaction continues for 15 hours at 12°C in a solution containing 66 mM tris-hydrochloride buffer (pH 7.6)-6.6 mM magnesium chloride-10 mM dithiothreitol-1 mM adenosine triphosphate. As a DNA recipient, E. coli K-12 strain JM103 is used. JM103 is a very popular strain that can be obtained simultaneously when, for example, pDR540 of Pharmacia is purchased. The organisms used in transformation are obtained by treating the culture in the logarithmic growth phase with calcium chloride at 0°C. Then obtained DNA-susceptible organisms are mixed with the ligated DNA sample and the mixture is let stand in ice for 20 minutes. After beating to 42°C for 2 minutes and keeping at 25°C for 10 minutes, the transformants are selected on an agar plate. The agar plate is composed of 1.5% agar (containing LB medium; 10 g trypton, 5 g yeast extract and 5 g sodium chloride in 1 liter) containing antibiotics kanamycin (50 µg/ml) and ampicillin (100 µg/ml). In this cloning, most of colonies formed must possess the intended plasmid, but plasmid is prepared from each colony and analyzed. Five colonies on the agar plate are transplanted on 5 ml of LB medium and incubated overnight at 37°C. From these bacteria, plasmids are prepared by the method of alkaline sodium dodecyl sulfate (SDS) (Birnboim, H.C. & Doly, J. Nucl. Acids Res. 7, 1513-1523, 1979) and subjected to double digestion at the HindIII site used in the cloning and EcoRI site of pUC13 and it is confirmed that a fragment of about 1.5 Kb is produced. Thus obtained plasmid (pUCKM) is digested with PstI, and of the DNAs isolated by agarose electrophoresis, only large fragment is recovered from the gel by the above method ad cyclized by using T4 DNA ligase. The transformant obtained thereby is selected on an agar plate (1.5% agar, LB medium) containing ampicillin (100 µg/ml) and the plasmid is prepared from the appearing colonies in the same manner as mentioned above and confirmed by agarose gel electrophoresis that two DNA bands appear by double digestion with HindIII and PstI and their sizes correspond to 2.7 Kb and 0.6 Kb. The intended plasmid (pUCKMF) obtained by this cloning can be a general expression plasmid for fused protein. A multiple cloning region of pUC13 is kept as it was at the downstream of SalI site of this plasmid, which is convenient when inserting a gene of an intended protein to express a fused protein. Besides, since the SalI site is also the restriction site of AccI and HincII, it is easy to match the reading frame of the protein encoded by the APH gene and the intended gene when fusing by utilizing the difference in the restriction style of three restriction enzymes and the subsequent repair by polymerase.

In constructing an expression vector for a fused protein with actin, pUCACT is used as a donor plasmid of DNA sequence (see Fig. 7) coding for the mature actin (Ohara, O. et al., Proceedings of the 23rd Meeting of Japan Society of Biophysics) (see Fig. 8). To begin with, mRNA is prepared from the hind leg muscle of rat by the SDS phenol method (Brawerman, G., Methods in Enzymology 30, 605-612, 1974) and a cDNA library is prepared by the Okayama-Berg method (Okayama, H. & Berg, P., Mol. Cell Biol. 2, 161-170, 1982). From this library the clone possessing the rat skeletal muscle actin cDNA is selected by the colony hybridization (Grunstein, M. & Hogness, D. S., Proc. Natl. Acad. Sci. U.S.A. 72, 3961-3965, 1975). The probe used in the screening is of β-actin gene (Wako Pure Chemical Industries, Ltd.). From the clones obtained by this screening, the plasmid is prepared by the alkaline SDS method. The sequence of the cDNA is estimated on the basis of the known base sequence of chromosomal gene of rat skeletal muscle actin (Nature 298, 857-859, 1982) and compared with the cDNA base sequence of the obtained clone. The base sequence is determined by using the M13 sequencing kit of Takara Shuzo Co., Ltd. As a result, thus obtained cDNA is confirmed to be a rat skeletal muscle actin cDNA of full length, from which pUCACT is constructed. To obtain a base sequence coding for the mature actin, the primer elongation method is employed. Rat actin cDNA in prepared an PstI fragment (about 1.4 Kb)and recovered by the agarose gel electrophoresis method mentioned above. This fragment and the M13 phage mp10 vector digested with PstI are subjected to ligation reaction by using T4 DNA ligase as stated above. By this ligation reaction product, strain JM103 is transformed and the transformant is mixed with 40 µl of 100 mM isopropyl-β-D-thiogalactopyranoside, 40 µl of 2% 5-bromo-4-chloro-3-indoryl-β-galactoside (dimethylformamide solution) and 250 µl of JM103 culture (LB medium). The mixture is added to 0.6% agar melted and kept at 42°C (TY medium: 8 g trypton, 5 g yeast extract and 5 g sodium chloride/liter), which is poured on a solidified 1.5% agar plate (TY medium). After the upper layer agar is solidified, it is kept overnight at 37°C. The organisms infected by M13 phage are retarded in the growth rate and form plaques. The recombinant phage into which the DNA fragment is inserted appears as a colorless plaque and the phage derived from M13 mp10 emerges as a blue plaque, so that the colorless plaque can be selected. An overnight culture of E. coli JM103 (1 ml) is planted into 100 ml of 2 × TY medium (16 g trypton, 10 g yeast extract and 5 g sodium chloride/liter) and, after 2-hour incubation, 5 ml each is dispensed into test tubes. Into each test tube, agar containing colorless plaque is inoculated by cutting it out with a capillary pipet. After incubation for further 5 hours at 37°C, the supernatant and the organisms are separated by centrifugation. From the organisms a replicative double-stranded DNA is prepared by the alkaline SDS method mentioned earlier and, from the supernatant, a phage single-stranded DNA is prepared. To the supernatant, about 800 µl of 20% polyethylene glycol 6000 and 2.5 M sodium chloride is added and the phages are collected by centrifugation. The phages are suspended in 10 mM tris-hydrochloride (pH 8.0)-1 mM EDTA, and a single-stranded DNA is prepared by phenol extraction and ethanol sedimentation. When the double-stranded DNA prepared from the colorless plaque is digested with BamHI, since there is BamHI site in the actin cDNA, a fragment of about 1000 bp or 400 bp is observed in agarose gel electrophoresis. This is caused by the reason that the actin cDNA can be inserted in to directions in this cloning, and what is intended is a phage DNA from which the fragment of about 1000 bp is produced. The single-stranded phage DNA prepared from the clone having the phage DNA which is regarded as the intended matter in this analysis is used in construction of pUCACT. Oligonucleotide possessing the base sequence coding for the amino terminal 5 residues of the mature actin is synthesized by the phosphotriester method (Nucl. Acids Res. 8, 5507-5517, 1980). The oligonucleotide 15-mer comprises a sequence of 5 GACGAGGACGAGACC3'. This oligonucleotide and the single-stranded phage DNA obtained by the above cloning are mused and then elongated with E. coli polymerase I Klenow fragment by using the single-stranded phage DNA as a template for 2 hours at 37°C in 10 mM tris-hydrochloride buffer (pH 7.5), 7 mM magnesium chloride, 20 mM sodium chloride and 0.2 mM each of 4 dNTP. After the reaction, the DNA is recovered by phenol extraction and ethanol sedimentation and the remaining portion in the single strand is digested with nuclease S1. After recovering the DNA by phenol extraction and ethanol sedimentation, the terminal is repaired to be flush by E. coli polymerase I Klenow fragment. The repair reaction is done in the same condition as the elongation reaction. After the reaction, the sample is treated for 20 minutes at 65°C to inactivate the Klenow fragment. Sodium chloride is added to the mixture so that the final concentration may be 100 mM and it is digested with PstI.

The digest is isolated by agarose gel electrophoresis and a DNA band of abut 1.2 Kb is recovered by using DEAE membrane. The product of pUC13 digested with both HincII and PstI and the above DNA fragment are mixed and ligated by using T4 DNA ligase. In the same manner as mentioned above the transformants are selected on the agar plate (1.5% agar, LB medium, ampicillin 100 µg/ml) and plasmids are prepared from arbitrary nine colonies by the alkaline SDS method. It is the intended plasmid that DNA band of about 1.2 Kb is generated in double digestion with HincII and EcoRI. In what is identified as the intended plasmid in this analysis, both HincII site and PstI site used in cloning are repaired. Also, a DNA fragment starting from the first base of the base sequence coding for the mature actin can be prepared by HincII digestion. By digesting this plasmid by HindIII, repairing with E. coli polymerase I Klenow fragment to make the terminal flush and then digesting with SalI, a DNA fragment of about 1.2 Kb is obtained, which is recovered from the agarose gel. The SalI-SmaI digest of pUC13 and the above DNA fragment are linked by using T4 DNA ligase. Same as stated above, plasmid is prepared from the transformant and it is the intended plasmid pUCACT that a DNA fragment of about 1.2 Kb is obtained by double digestion with EcoRI-HincII.

A fragment of about 600 bp obtained by double digestion of pUCKMF with HindIII-HincII and a fragment of about 3.9 Kb obtained by digestion of pUCACT with HindIII-HincII are recovered from agarose gel and ligated by using T4 DNA ligase. In the same way as described before, the transformant (JM103) is selected on an agar plate containing ampicillin and the plasmid is prepared from about 10 colonies. It is the intended plasmid that a fragment of about 1.5 Kb is produced by double digestion with HindIII-EcoRI. The obtained intended plasmid (pUCKMFACT) possesses a sequence encoding a polypeptide chain with a total of 446 residues comprising 61 amino acid residues derived from APH and 10 amino acid residues derived from transposon Tn5 at the amino terminal and, thereafter, 375 residues of actin (see Fig. 9).

For the expression of fused actin, firstly, E. coli carrying the above pUCKMACT is incubated in LB medium containing 40 µg/ml of ampicillin for 14 hours at 37 °C. This culture (100 µl) is added to 5 ml of M9 medium (6 g $Na_2HPO_4$, 3 g $KH_2PO_4$, 0.5 g NaCl, 1 g $NH_4Cl$/liter, 2 mM $MgSO_4$, 0.1 mM $CaCl_2$, 0.5% glycerol, 0.5% casamino acid and ampicillin 40 µg/ml) and further incubated for 10 hours at 37 °C. Two hours after incubation, isopropyl-β-D-thiogalactopyranoside is added by 1 mM for induction. After incubation the organisms are collected by centrifugation and suspended in 10% glycerol-0.1 M tris-hydrochloride buffer-1% SDS-1% 2-mercaptoethanol. After ultrasonically treated and heated to 100 °C for 2 minutes, the resultant is analyzed by polyacrylamide gel electrophoresis in the presence of SDS according to the method proposed by Laemmli et al. (Nature 227, 680-683, 1970).

In the pattern of SDS polyacrylamide gel electrophoresis, a clear band was observed at a position nearly corresponding to the molecular weight of the intended fused actin (about 15% of the total bacterial protein). This band was confirmed to be the intended object because the protein in this band can be bonded with monoclonal antibody against actin (Amersham International).

The DNA fragment of about 400 bp obtained by double digestion of the pUCKMF by HindIII-HaeIII is linked with the DNA obtained by digesting pUCACT with SalI, treating with E. coli polymerase I Klenow fragment and further digesting with HindIII, thereby obtaining a expression plasmid pUCKMFACT4 for fused actin comprising 12 residues of amino terminal of APH and 1 residue as the connecting portion (a total of 13 residues) followed by actin. When the fused protein was produced by using E. coli having this plasmid in the same way as mentioned above, an obvious band was not observed on the gel (less than 1% of the total bacterial protein).

An expression plasmid for mature actin itself using pUC vector was built up according to the principle of two-cistron construction reported by Schoner et al. (Proc. Natl. Acad. Sci. USA 81, 5403-5407, 1984) (see Figs. 9, 10 and 11). In the construction according to this principle, the termination codon TAA, ribosome-binding sequence and initiation codon ATG for start of translation of the following actin gene are inserted between the region encoding 60 residues of amino terminal of APH and the actin gene. This is intended to express mature actin without sacrificing the high translation efficiency of the structure derived from upstream APH gene and successful expressions of several genes by the similar methods have been reported (EP-154539).

The pUCKMF is digested with PstI, of which the terminal is made flush by E. coli polymerase I Klenow fragment. To the flush ends a XbaI line (5'-phosphate linker, 5'-CTCTAGAG-3'; Pharmacia) is linked with T4 DNA ligase according to the conventional method (Molecular Cloning, Cold Spring Harbor Laboratory, New York, 1982). The DNA to which XbaI linkers are added is digested with XbaI. After XbaI digestion the DNA is recovered by phenol extraction and ethanol sedimentation. The both ends of the obtained linear DNA are linked with T4 DNA ligase so as to form a closed circular DNA. After the ligation the DNA is introduced into E. coli JM103 in the same manner as described above and the plasmid is prepared from the obtained transformant. Then, it is confirmed that the inserted fragment of about 600 bp is generated by HindIII and XbaI digestion and that there is no PstI restriction site. This plasmid pUCKMF1 can be used not only as expression plasmid by two-cistron construction but also in construction of expression plasmid of fused actin (see Fig. 10).

This pUCKMF1 is treated with XbaI and then with Klenow fragment and further digested with EcoRI. Thus obtained DNA fragment is linked by means of T4 DNA ligase with the DNA fragment containing the actin-coding region of about 1.2 Kb, which is produced by digestion of pUCACT with EcoRI after treatment with SalI and Klenow fragment, to obtain a expression plasmid for fused actin. This fused actin-expression plasmid (pUCKMFACT1) expresses actin fused with 60 amino acid residues of amino terminal of APH and 3 amino acid residues as the connecting portion (see Fig. 11).

For the construction of mature actin-expression plasmid by two-cistron construction, a synthetic oligonucleotide is inserted into the XbaI-SstI site of pUC vector containing the APH gene to which XbaI linkers are added as mentioned above, so that the base sequence as shown in Fig. 13 is included at the downstream of the base sequence derived from the APH gene. Thus obtained vector can be used as ATG vector as reported by Nishi et al. (DNA 2, 265-273 1983). That is, by a proper enzyme treatment, it may be used as a donor vector of promoter and a base sequence necessary for initiation of translation including the initiation codon. This vector is digested with SstI and the terminal is made flush by Klenow fragment, followed by further digestion with EcoRI. This DNA is linked with the DNA fragment including the region coding for actin of about 1.2 Kb obtained by digestion of pUCACT with HincII-EcoRI, by using T4 DNA ligase, to give an expression plasmid pUCKMACT for mature actin conforming to the two-cistron construction (see Fig. 12). It was identified as the intended plasmid by confirming that a fragment of about 1.8 Kb is produced by HindIII-EcoRI digestion of the plasmid obtained from the transformant and by checking the base sequence in the vicinity of initiation codon ATG of actin by means of sequencing kit manufactured by Takara Shuzo Co., Ltd.

Changes in production output by length of APH gene

To see how many residues of the amino terminal of APH used in fusion are necessary for high expression, the change of the production of fused actin accompanied with the shortening of the fusion part of APH by exonuclease BAL 31 from the HincII site of pUCKMACT was investigated and it has been found that the fused actin is produced in above 10% of the total bacterial protein when at least 25 residues of APH amino terminal are fused.

The pUCKMF cut by SalI is digested with exonuclease BAL31. The digestion with BAL31 is done in 20 mM tris-hydrochloride buffer (pH 8.0), 12 mM calcium chloride 12 mM magnesium chloride and 0.6 M sodium chloride and the reaction is stopped by adding EDTA so that the final concentration becomes 50 mM. The length shortened by BAL31 treatment is adjusted by the digestion time. The DNA digested with BAL31 is further treated by E. coli polymerase I Klenow fragment to make the terminal flush. After digestion with HindIII, fragments of about 400 to 600 bp are recovered from agarose gel. After digesting pUCACT with SalI, it is treated with E. coli polymerase I Klenow fragment and Hind III. The resulting plasmid and the BAL31 treated DNA as mentioned above are linked together by using T4 DNA ligase. By the resulting DNA, E. coli JM103 is transformed in the same manner as described above and the transformants are selected on an agar plate containing ampicillin. After incubating each colony, plasmids are prepared from about 40 colonies. From the size of the DNA fragment obtained by BglII digestion, the DNA length shortened by BAL31 is approximately calculated. A clone having the intended DNA of shortened length is selected and the base sequence at the connection portion between actin and APH genes is determined by using a sequencing kit of Takara Shuzo Co., Ltd. In this way, the expression plasmid of actin having the same reading frame as the coding region of actin and also possessing the shortened APH gene region is isolated. In this way, pUCKMFACT2 expressing rat actin after 44 amino acid residues of APH and 1 amino acid residue of connecting sequence and pUCKMFACT3 expressing rat actin after 24 amino acid residues of APH and 1 amino acid residue of connecting sequence were obtained.

The fused proteins expressed by thus obtained vectors pUCKMFACT, pUCKMFACT1, pUCKMFACT2, pUCKMFACT3, pUCKMFACT4 and pUCKMACT (however, pUCKMACT expresses mature rat actin) are shown in Fig. 14.

Fig. 15 shows the results of analysis of production output of each fused protein by polyacrylamide gel electrophoresis of the protein, which is extracted and solubilized by incubating E. coli holding each plasmid and breaking it in the presence of sodium dodecyl sulfate. The quantity of each fused protein was determined from the quantity of monoclonal anti-actin antibody binding to fused actin after transferring it into a nitrocellulose membrane or from staining band by Coomassie Brilliant Blue-R250.

While the fused portion of APH dies from 25 amino acid residues to 13 amino acid residues (actually, since 1 amino acid residue is contained as the connecting part, the amino terminal residues of APH are respectively 24 and 12), a significant decrease in the production is observed. This fused actin lowered in the production is, however, expressed 5 to 10 times as much as mature actin expressed by pUCKMACT.

**Claims**

1. A method for preparing a protein comprising introducing into a microorganism a vector capable of expressing the protein in said microorganism as a fused protein with APH, cultivating said microorganism to produce said fused protein and recovering said protein from said fused protein, wherein said APH comprises 13 to 61 N-terminal amino acid residues of the APH amino acid sequence shown in Fig. 1.

2. The method of claim 1, wherein said APH comprises 24 to 61 N-terminal amino acid residues of the APH amino acid sequence shown in Fig. 1.

3. The method claimed in any one of claims 1 or 2, wherein said fused protein has a binding sequence between APH and said protein which comprises at least one amino acid residue.

4. The method claimed in claim 3, wherein said binding sequence is methionine or a sequence containing methionine.

5. The method claimed in any one of claims 1-4, wherein said protein is human PSTI or rat actin.

6. The method claimed in any one of claims 1-5, wherein said microorganism is Escherichia coli.

**Patentansprüche**

1. Verfahren zur Herstellung eines Proteins, umfassend das Einführen eines Vektors in einen Mikroorganismus, wobei der Vektor das Protein in dem Mikroorganismus als Fusionsprotein mit APH exprimieren kann, Züchtung des Mikroorganismus zur Herstellung des Fusionsproteins und Gewinnung des Proteins aus dem Fusionsprotein, wobei die APH 13 bis 61 N-terminale Aminosäurereste der APH-Aminosäuresequenz gemäß Fig. 1 umfaßt.

2. Verfahren nach Anspruch 1, wobei die APH 24 bis 61 N-terminale Aminosäurereste der APH-Aminosäuresequenz gemäß Fig. 1 umfaßt.

3. Verfahren nach einem der Ansprüche 1 oder 2, wobei das Fusionsprotein eine Bindungssequenz zwischen APH und dem Protein aufweist, die mindestens einen Aminosäurerest umfaßt.

4. Verfahren nach Anspruch 3, wobei die Bindungssequenz Methionin oder eine Methionin enthaltende Sequenz ist.

5. Verfahren nach einem der Ansprüche 1 bis 4, wobei das Protein menschliches PSTI oder Ratten-Actin ist.

6. Verfahren nach einem der Ansprüche 1 bis 5, wobei der Mikroorganismus Escherichia coli ist.

**Revendications**

1. Procédé de préparation d'une protéine comprenant une introduction dans un micro-organisme d'un vecteur capable d'exprimer la protéine dans ce microorganisme sous la forme d'une protéine fusionnée avec du APH, une culture de ce micro-organisme pour produire cette protéine fusionnée et une récupération de cette protéine à partir de la protéine fusionnée, ledit APH comprenant 13 à 61 restes d'aminoacide à N terminal de la séquence d'aminoacides d'APH représentée sur la figure 1.

2. Procédé suivant la revendication 1, caractérisé en ce que ledit APH comprend 24 à 61 restes d'aminoacide à N terminal de la séquence d'aminoacides d'APH représentée sur la figure 1.

3. Procédé suivant l'une ou l'autre des revendications 1 et 2, caractérisé en ce que la protéine fusionnée a une séquence de liaison entre l'APH et ladite protéine qui comprend au moins un reste d'aminoacide.

4. Procédé suivant la revendication 3, caractérisé en ce que la séquence de liaison est de la méthionine ou une séquence contenant de la méthionine.

5. Procédé suivant l'une quelconque des revendications 1 à 4, caractérisé en ce que ladite protéine est du PSTI humain ou de l'actine de rat.

6. Procédé suivant l'une quelconque des revendications 1 à 5, caractérisé en ce que le microorganisme est Escherichia coli.

Fig. 1

```
                              -450      -440      -430
                              GAGCCGAGAGGCAACGCATGGAGGAGCCGG
      -410      -400      -390      -380      -370
ATAATCTGGAGCGGATGGTCTCGATCCTCTCGTTTGTTGCGGTCAGGCTGTTACAGCTCA
      -350      -340      -330      -320      -310
GAGAAAGCTTCACGCTGCCGCAAGCACTCAGGGCGCAAGGGCTGCTAAAGGAAGCGGAAC
      -290      -280      -270      -260      -250
ACGTAGAAAGCCAGTCCGCAGAAACGGTGCTGACCCCGGATGAATGTCAGCTACTGGGCT
      -230      -220      -210      -200      -190
ATCTGGACAAGGGAAAACGCAAGCGCAAAGAGAAAGCAGGTAGCTTGCAGTGGGCTTACA
      -170      -160      -150      -140      -130
TGGCGATAGCTAGACTGGGCGGTTTTATGGACAGCAAGCGAACCGGAATTGCCAGCTGGG
      -110      -100       -90       -80       -70
GCGCCCTCTGGTAAGGTTGGGAAGCCCTGCAAAGTAAACTGGATGGCTTTCTTGCCGCCA
       -50       -40       -30       -20       -10        -1
AGGATCTGATGGCGCAGGGGATCAAGATCTGATCAAGAGACAGGATGAGGATCGTTTCGC

1        10        20        30        40        50        60
ATGATTGAACAAGATGGATTGCACGCAGGTTCTCCGGCCGCTTGGGTGGAGAGGCTATTC
M  I  E  Q  D  G  L  H  A  G  S  P  A  A  W  V  E  R  L  F

         70        80        90       100       110       120
GGCTATGACTGGGCACAACAGACAATCGGCTGCTCTGATGCCGCCGTGTTCCGGCTGTCA
G  Y  D  W  A  Q  Q  T  I  G  C  S  D  A  A  V  F  R  L  S

        130       140       150       160       170       180
GCGCAGGGGCGCCCGGTTCTTTTTGTCAAGACCGACCTGTCCGGTGCCCTGAATGAACTG
A  Q  G  R  P  V  L  F  V  K  T  D  L  S  G  A  L  N  E  L
```

Fig. 1 (continued)

```
       190       200       210       220       230       240
CAGGACGAGGCAGCGCGGCTATCGTGGCTGGCCACGACGGGCGTTCCTTGCGCAGCTGTG
Q  D  E  A  A  R  L  S  W  L  A  T  T  G  V  P  C  A  A  V

       250       260       270       280       290       300
CTCGACGTTGTCACTGAAGCGGGAAGGGACTGGCTGCTATTGGGCGAAGTGCCGGGGCAG
L  D  V  V  T  E  A  G  R  D  W  L  L  L  G  E  V  P  G  Q

       310       320       330       340       350       360
GATCTCCTGTCATCTCACCTTGCTCCTGCCGAGAAAGTATCCATCATGGCTGATGCAATG
D  L  L  S  S  H  L  A  P  A  E  K  V  S  I  M  A  D  A  M

       370       380       390       400       410       420
CGGCGGCTGCATACGCTTGATCCGGCTACCTGCCCATTCGACCACCAAGCGAAACATCGC
R  R  L  H  T  L  D  P  A  T  C  P  F  D  H  Q  A  K  H  R

       430       440       450       460       470       480
ATCGAGCGAGCACGTACTCGGATGGAAGCCGGTCTTGTCGATCAGGATGATCTGGACGAA
I  E  R  A  R  T  R  M  E  A  G  L  V  D  Q  D  D  L  D  E

       490       500       510       520       530       540
GAGCATCAGGGGCTCGCGCCAGCCGAACTGTTCGCCAGGCTCAAGGCGCGCATGCCCGAC
E  H  Q  G  L  A  P  A  E  L  F  A  R  L  K  A  R  M  P  D

       550       560       570       580       590       600
GGCGAGGATCTCGTCGTGACCCATGGCGATGCCTGCTTGCCGAATATCATGGTGGAAAAT
G  E  D  L  V  V  T  H  G  D  A  C  L  P  N  I  M  V  E  N
```

Fig. 1 (continued)

```
       190                 200                 210                 220                 230                 240
CAGGACGAGGCAGCGCGGCTATCGTGGCTGGCCACGACGGGCGTTCCTTGCGCAGCTGTG
Q  D  E  A  A  R  L  S  W  L  A  T  T  G  V  P  C  A  A  V

       250                 260                 270                 280                 290                 300
CTCGACGTTGTCACTGAAGCGGGAAGGGACTGGCTGCTATTGGGCGAAGTGCCGGGGCAG
L  D  V  V  T  E  A  G  R  D  W  L  L  L  G  E  V  P  G  Q

       310                 320                 330                 340                 350                 360
GATCTCCTGTCATCTCACCTTGCTCCTGCCGAGAAAGTATCCATCATGGCTGATGCAATG
D  L  L  S  S  H  L  A  P  A  E  K  V  S  I  M  A  D  A  M

       370                 380                 390                 400                 410                 420
CGGCGGCTGCATACGCTTGATCCGGCTACCTGCCCATTCGACCACCAAGCGAAACATCGC
R  R  L  H  T  L  D  P  A  T  C  P  F  D  H  Q  A  K  H  R

       430                 440                 450                 460                 470                 480
ATCGAGCGAGCACGTACTCGGATGGAAGCCGGTCTTGTCGATCAGGATGATCTGGACGAA
I  E  R  A  R  T  R  M  E  A  G  L  V  D  Q  D  D  L  D  E

       490                 500                 510                 520                 530                 540
GAGCATCAGGGGCTCGCGCCAGCCGAACTGTTCGCCAGGCTCAAGGCGCGCATGCCCGAC
E  H  Q  G  L  A  P  A  E  L  F  A  R  L  K  A  R  M  P  D

       550                 560                 570                 580                 590                 600
GGCGAGGATCTCGTCGTGACCCATGGCGATGCCTGCTTGCCGAATATCATGGTGGAAAAT
G  E  D  L  V  V  T  H  G  D  A  C  L  P  N  I  M  V  E  N
```

Fig. 2

```
1                                        10                                         20
AspSerLeuGlyArgGluAlaLysCysTyrAsnGluLeuAsnGlyCysThrLysIleTyr
GACTCCCTGGGAAGAGAGGCCAAATGTTACAATGAACTTAATGGATGCACCAAGATATAT
                             30                             60

                             30                             40
AspProValCysGlyThrAspGlyAsnThrTyrProAsnGluCysValLeuCysPheGlu
GACCCTGTCTGTGGGACTGATGGAAATACTTATCCCAATGAATGCGTGTTATGTTTTGAA
                             90                            120

                             50
AsnArgLysArgGlnThrSerIleLeuIleGlnLysSerGlyProCys***
AATCGGAAACGCCAGACTTCTATCCTCATTCAAAAATCTGGGCCTTGCTGA
                             150
```

Fig. 3

```
      1                                       10                                          20
   MetAspSerLeuGlyArgGluAlaLysCysTyrAsnGluLeuAsnGlyCysThrLysIleTyr
<----U-1----><----U-2----><----U-3----><----U-4---->
CGACATGGACTCCCTGGGAAGAGAGGCCAAATGTTACAATGAACTTAATGGATGCACCAAGATATATG
  TGTACCTGAGGGACCCTTCTCTCCGGTTTACAATGTTACTTGAATTACCTACGTGGTTCTATATACTGGGACAGA
  <----L-1----><----L-2----><----L-3----><----L-4---->
AccI

                                         30
AspProValCysGlyThrAspGlyAsnThrTyrProAsnGluCysValLeuCysPhe
 <----U-5----><----U-6----><----U-7---->
 ACCCTGTCTGTGGGACTGATGGAAATACTTATCCCAATGAATGCGTGTTATGTTTT
           CACCCTGACTACCTTTATGAATAGGGTTACTTACGCACAATACAAAACTTTTAGCC
           <----L-5----><----L-6----><----L-7---->

40                                          50
GluAsnArgLysArgGlnThrSerIleLeuIleGlnLysSerGlyProCys***
<----U-8----><----U-9----><----U-10---->
GAAAATCGGAAACGCCAGACTTCTATCCTCATTCAAAAATCTGGGCCTTGCTGAG
          TTTGCGGTCTGAAGATAGGAGTAAGTTTTTAGACCCGGAACGACTCCTAG
          <----L-8----><----L-9----><----L-10---->
                                                            BamHI
```

Fig. 4

RESTRICTION MAP OF THE APH GENE AND ITS FLANKING REGIONS

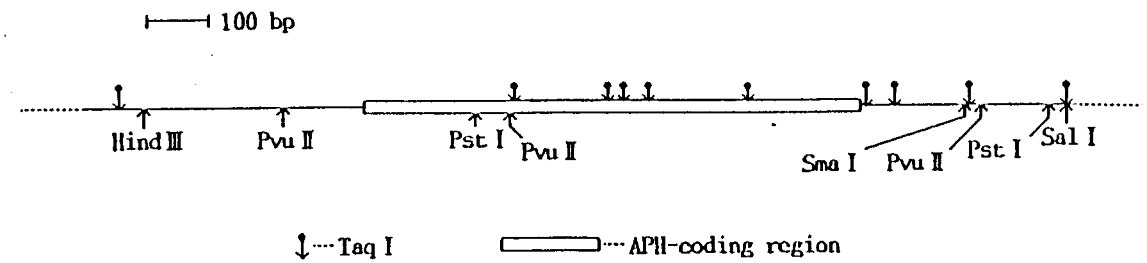

Fig. 5

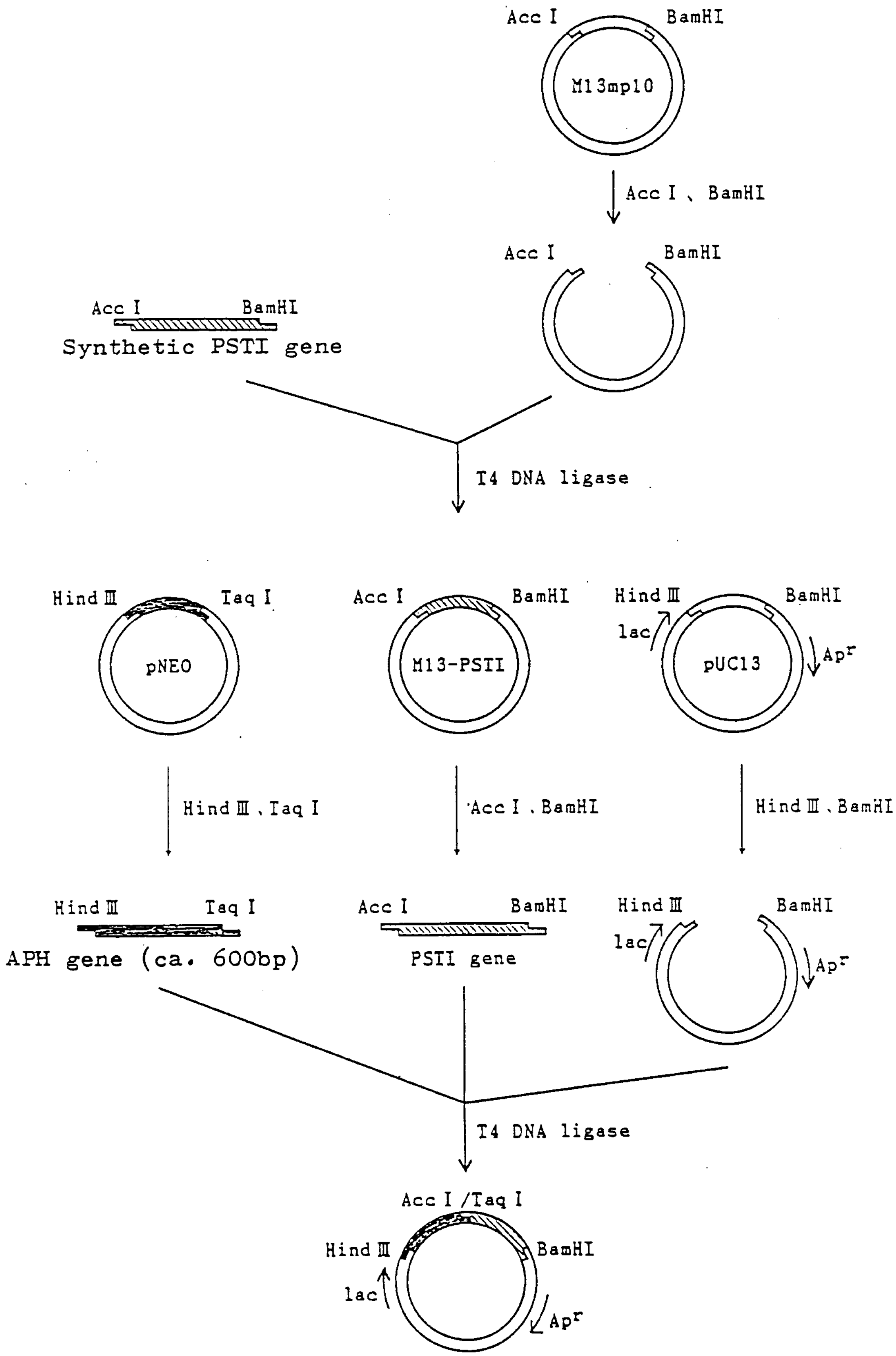
Acc I
BamHI
M13mp10
Acc I 、BamHI
Acc I
BamHI
Acc I
BamHI
Synthetic PSTI gene
T4 DNA ligase
Hind III
Taq I
pNEO
Acc I
BamHI
M13-PSTI
Hind III
BamHI
lac
pUC13
Apr
Hind III 、Taq I
Acc I 、BamHI
Hind III 、BamHI
Hind III
Taq I
APH gene (ca. 600bp)
Acc I
BamHI
PSTI gene
Hind III
BamHI
lac
Apr
T4 DNA ligase
Acc I /Taq I
Hind III
BamHI
lac
Apr

Fig. 6

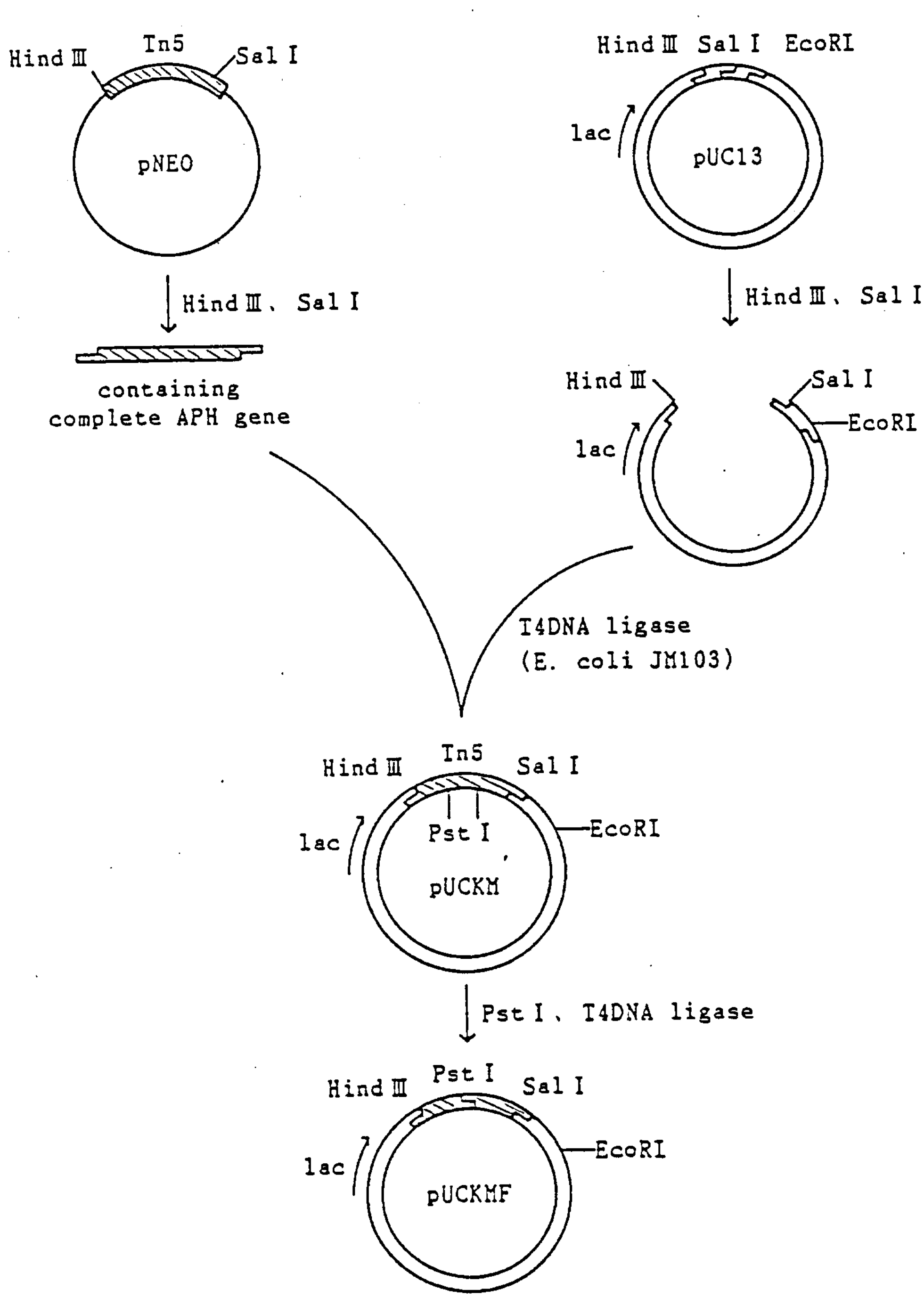
Hind III
Tn5
Sal I
pNEO
Hind III Sal I EcoRI
lac
pUC13
Hind III、 Sal I
Hind III、 Sal I
containing
complete APH gene
Hind III
Sal I
EcoRI
lac
T4DNA ligase
(E. coli JM103)
Hind III
Tn5
Sal I
lac
Pst I
EcoRI
pUCKM
Pst I、 T4DNA ligase
Hind III
Pst I
Sal I
EcoRI
lac
pUCKMF

Fig. 7

```
CACACGGACGTGAAGCCTCACTTCCTACCCTCGGCACCAGGGCCAGAGTCAGAGCAGCAGGAAACTAGACACC

         10        20        30        40        50        60        70
ATGTGTGACGAGGACGAGACCACCGCTCTTGTGTGTGACAACGGCTCTGGCCTGGTGAAAGCTGGCTTTGCCGGG
M  C  D  E  D  E  T  T  A  L  V  C  D  N  G  S  G  L  V  K  A  G  F  A  G

         85        95       105       115       125       135       145
GATGATGCCCCCAGGGCTGTGTTCCCATCCATCGTGGGTCGGCCCCGTCACCAGGGTGTCATGGTCGGTATGGGT
D  D  A  P  R  A  V  F  P  S  I  V  G  R  P  R  H  Q  G  V  M  V  G  M  G

        160       170       180       190       200       210       220
CAGAAGGATTCCTACGTGGGCGACGAGGCCCAGAGCAAGCGAGGTATCCTGACCCTGAAGTACCCCATTGAACAC
Q  K  D  S  Y  V  G  D  E  A  Q  S  K  R  G  I  L  T  L  K  Y  P  I  E  H

        235       245       255       265       275       285       295
GGCATTATCACCAACTGGGACGACATGGAGAAGATCTGGCACCACACCTTCTACAATGAGCTGCGTGTGGCCCCC
G  I  I  T  N  W  D  D  M  E  K  I  W  H  H  T  F  Y  N  E  L  R  V  A  P

        310       320       330       340       350       360       370
GAGGAGCACCCGACCCTGCTCACTGAGGCCCCCTTGAACCCCAAAGCTAACCGGGAGAAGATGACTCAAATCATG
E  E  H  P  T  L  L  T  E  A  P  L  N  P  K  A  N  R  E  K  M  T  Q  I  M

        385       395       405       415       425       435       445
TTTGAGACCTTCAACGTGCCTGCTATGTATGTGGCTATTCAGGCGGTGCTGTCTCTCTATGCCTCCGGCCGAACC
F  E  T  F  N  V  P  A  M  Y  V  A  I  Q  A  V  L  S  L  Y  A  S  G  R  T
```

Fig. 7 (continued)

```
460 470 480 490 500 510 520
ACCGGCATCGTGTTGGATTCTGGGGACGGTGTCACCCACAACGTGCCCATCTATGAGGGTTATGCCCTGCCACAC
T G I V L D S G D G V T H N V P I Y E G Y A L P H

535 545 555 565 575 585 595
GCCATCATGCGTCTGGACCTCGCTGGTCGCGACCTTACTGACTACCTGATGAAAATCCTCACCGAGCGTGGCTAT
A I M R L D L A G R D L T D Y L M K I L T E R G Y

610 620 630 640 650 660 670
TCCTTCGTGACCACAGCTGAACGTGAGATTGTGCGCGACATCAAAGAGAAGCTGTGCTACGTGGCCCTGGACTTC
S F V T T A E R E I V R D I K E K L C Y V A L D F

685 695 705 715 725 735 745
GAGAATGAGATGGCCACCGCTGCCTCTTCCTCCTCCCTGGAAAAGAGCTATGAGCTGCCTGACGGGCAGGTCATC
E N E M A T A A S S S S L E K S Y E L P D G Q V I

760 770 780 790 800 810 820
ACCATCGGCAATGAGCGCTTCCGTTGCCCGGAGACGCTCTTCCAGCCTTCCTTTATCGGTATGGAGTCTGCGGGG
T I G N E R F R C P E T L F Q P S F I G M E S A G

835 845 855 865 875 885 895
ATCCATGAGACCACCTACAACAGCATCATGAAGTGCGACATCGACATCAGGAAGGACCTGTACGCCAACAACGTC
I H E T T Y N S I M K C D I D I R K D L Y A N N V
```

Fig. 7 (continued)

```
        910       920       930       940       950       960       970
ATGTCAGGGGGCACTACCATGTACCCCGGTATCGCTGACCGCATGCAGAAGGAGATCACAGCTCTGGCTCCCAGC
M  S  G  G  T  T  M  Y  P  G  I  A  D  R  M  Q  K  E  I  T  A  L  A  P  S

        985       995      1005      1015      1025      1035      1045
ACCATGAAGATCAAGATCATCGCCCCCCCTGAGCGCAAGTACTCAGTGTGGATCGGCGGCTCCATCCTGGCCTCG
T  M  K  I  K  I  I  A  P  P  E  R  K  Y  S  V  W  I  G  G  S  I  L  A  S

       1060      1070      1080      1090      1100      1110      1120
CTGTCCACCTTCCAGCAGATGTGGATCACCAAGCAGGAGTACGACGAGGCCGGCCCCTCCATTGTGCACCGCAAA
L  S  T  F  Q  Q  M  W  I  T  K  Q  E  Y  D  E  A  G  P  S  I  V  H  R  K

TGCTTCTAGGCGCACCGCGTCTGTGTACGCGCTCTCTCTCCTCAGGACGACAATCGACCATCGTGCTATGGTTGC
C  F  *

AGGGTGGCCCCATCCTCCGCGTGGCTCCATCGCCGCCACTGCAGCCGGCGCCTGTTTTTGACGTGTACATAGATT

CGCTCGTTTTACCTCATTTTGTTATTTTTCAAACAAAGCCCTGTGGAAGGAAATGGAAAACTTGAAGCATTAAAG

CAGCCATTCTGTTTTGCTCCAAAAAAAAAAAAAAAAAAAAAAAAAAAAAAAAAAAAAAAA
```

Fig. 8

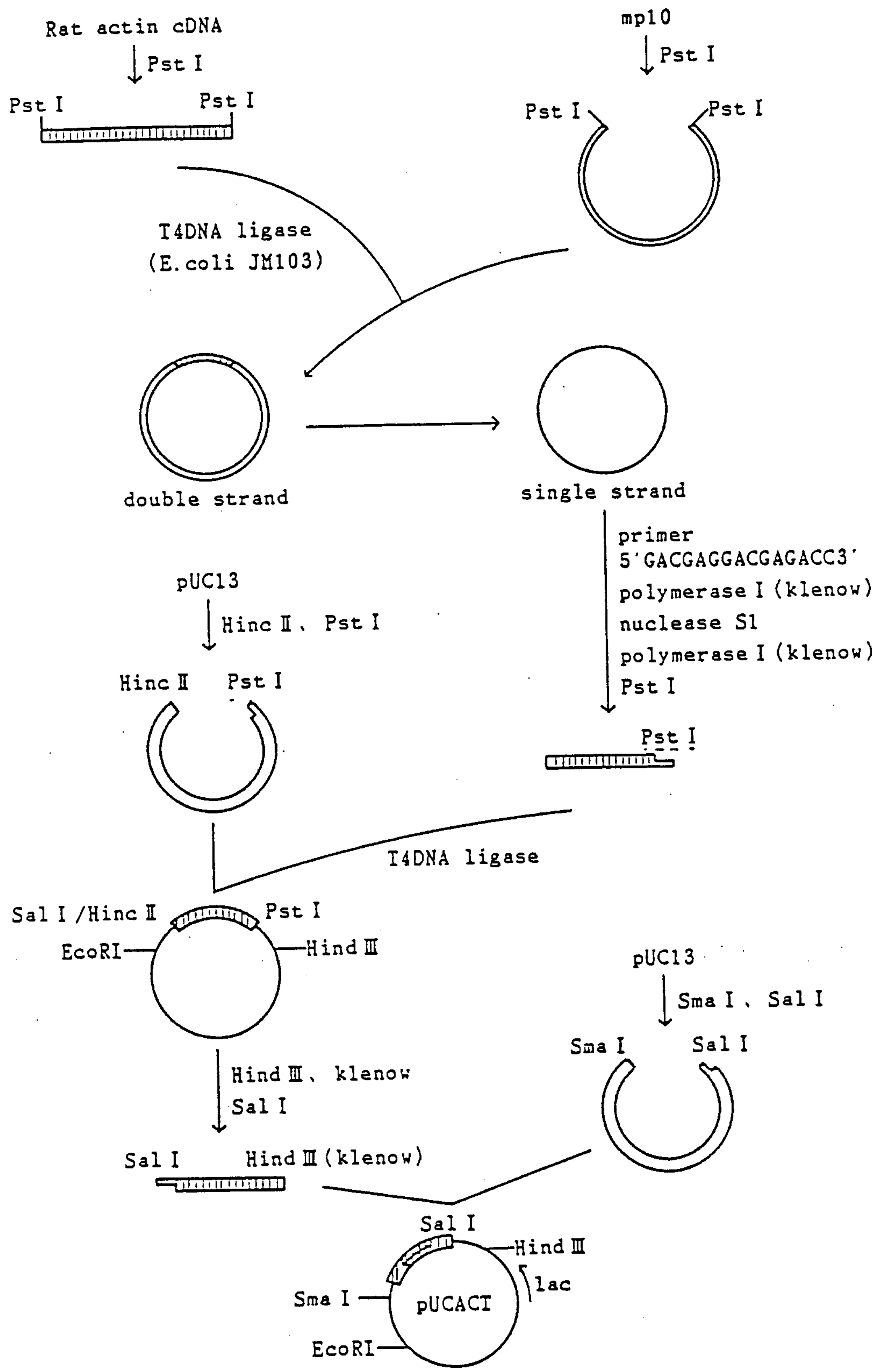
Rat actin cDNA
Pst I
Pst I
Pst I
mp10
Pst I
Pst I
Pst I
T4DNA ligase
(E.coli JM103)
double strand
single strand
primer
5'GACGAGGACGAGACC3'
polymerase I (klenow)
nuclease S1
polymerase I (klenow)
Pst I
pUC13
Hinc II、 Pst I
Hinc II
Pst I
Pst I
T4DNA ligase
Sal I /Hinc II
Pst I
EcoRI
Hind III
pUC13
Sma I 、 Sal I
Sma I
Sal I
Hind III、 klenow
Sal I
Sal I
Hind III (klenow)
Sal I
Hind III
lac
Sma I
pUCACT
EcoRI

Fig. 9

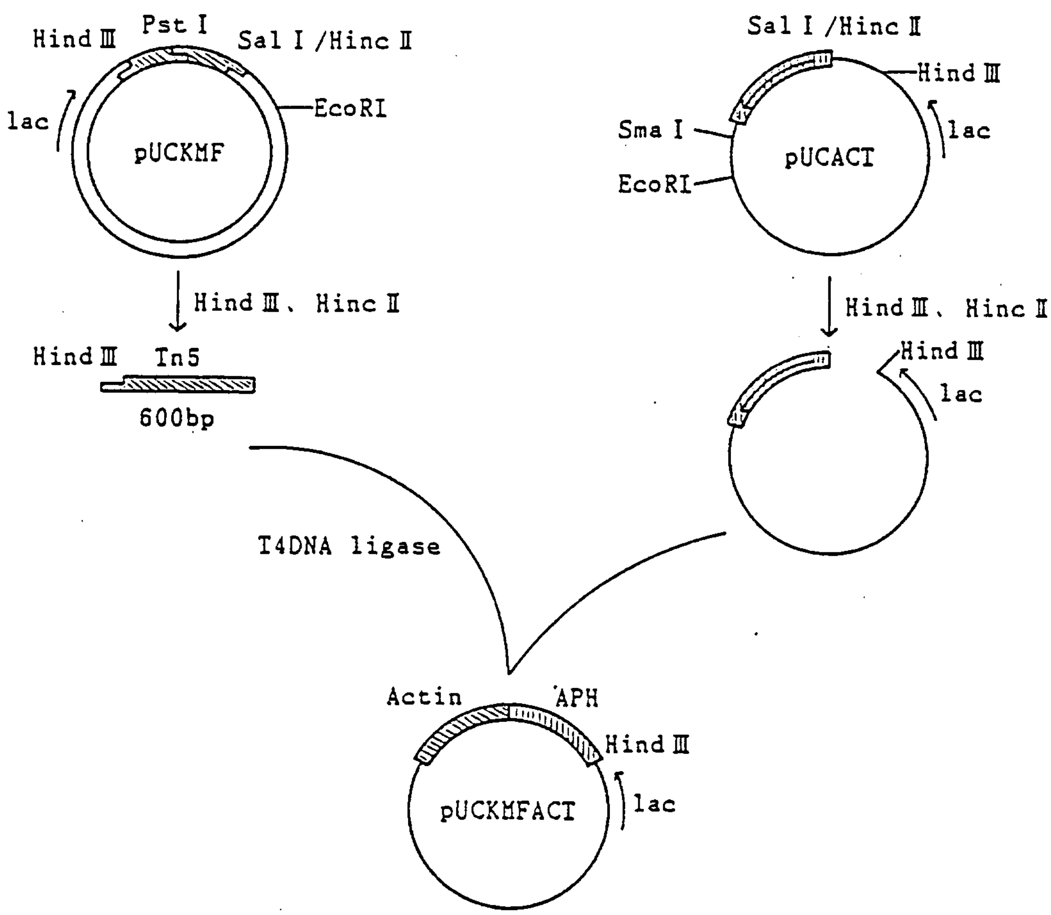
Hind Ⅲ
Pst I
Sal I /Hinc Ⅱ
lac
EcoRI
pUCKMF
Hind Ⅲ、Hinc Ⅱ
Hind Ⅲ
Tn5
600bp
Sal I /Hinc Ⅱ
Hind Ⅲ
Sma I
lac
pUCACT
EcoRI
Hind Ⅲ、Hinc Ⅱ
Hind Ⅲ
lac
T4DNA ligase
Actin
APH
Hind Ⅲ
pUCKMFACT
lac

Fig. 10

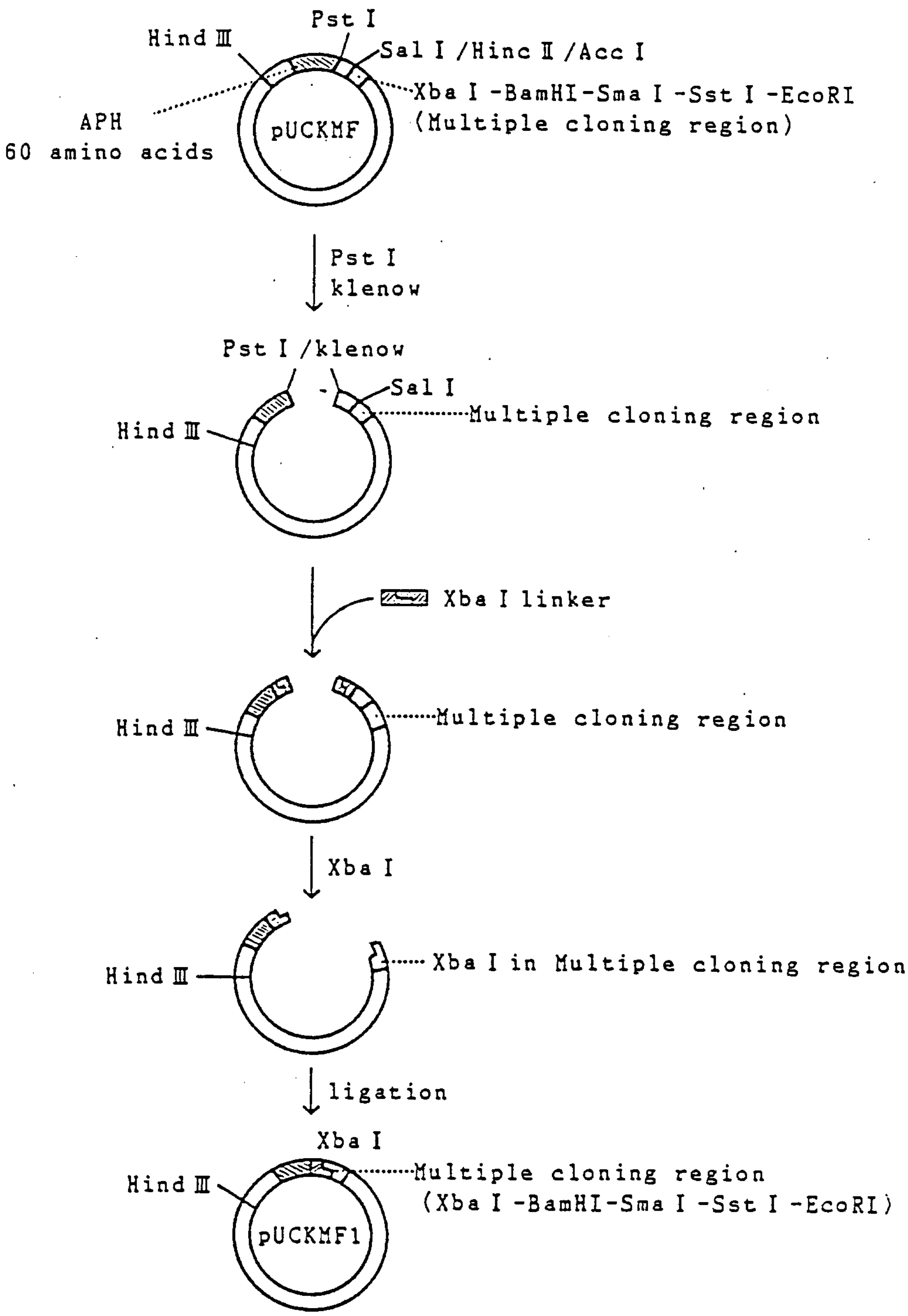
Pst I
Hind III
Sal I /Hinc II /Acc I
Xba I -BamHI-Sma I -Sst I -EcoRI
(Multiple cloning region)
APH
60 amino acids
pUCKMF
Pst I
klenow
Pst I /klenow
Sal I
Hind III
Multiple cloning region
Xba I linker
Hind III
Multiple cloning region
Xba I
Hind III
Xba I in Multiple cloning region
ligation
Xba I
Hind III
Multiple cloning region
(Xba I -BamHI-Sma I -Sst I -EcoRI)
pUCKMF1

Fig. 11

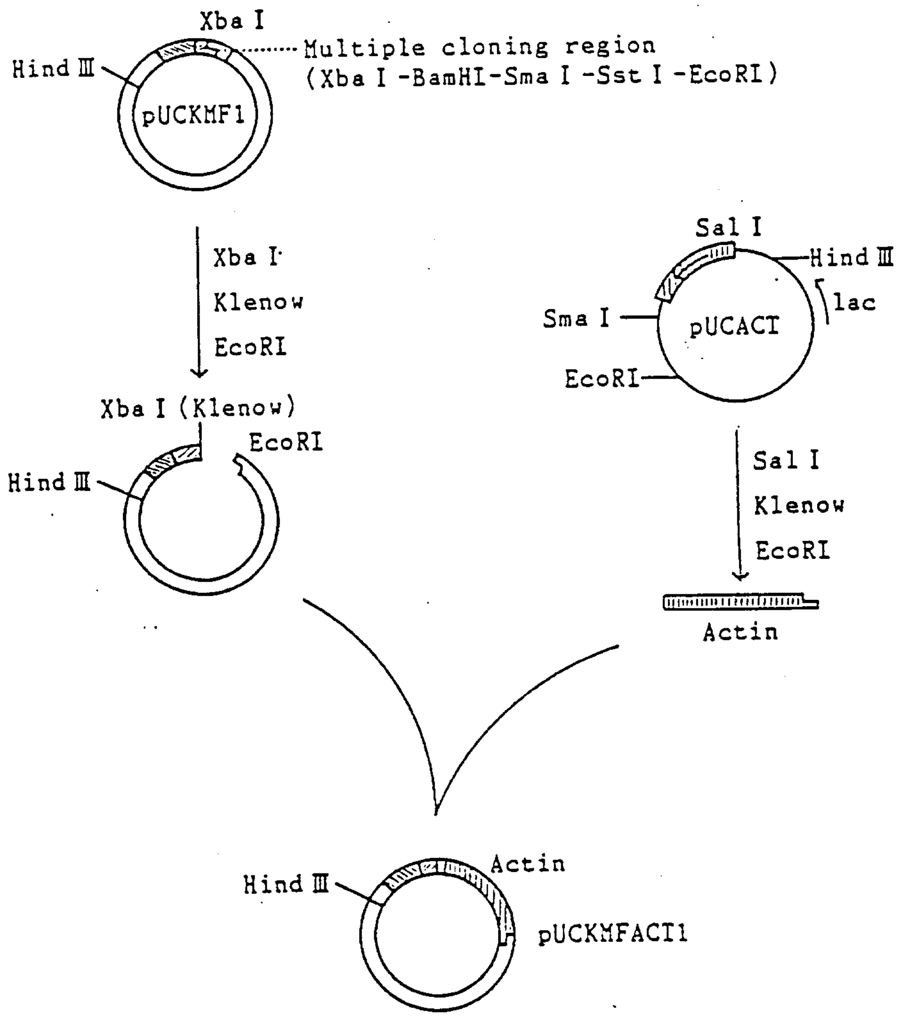


APH 60 res. + Junction 3 res.

Fig. 12

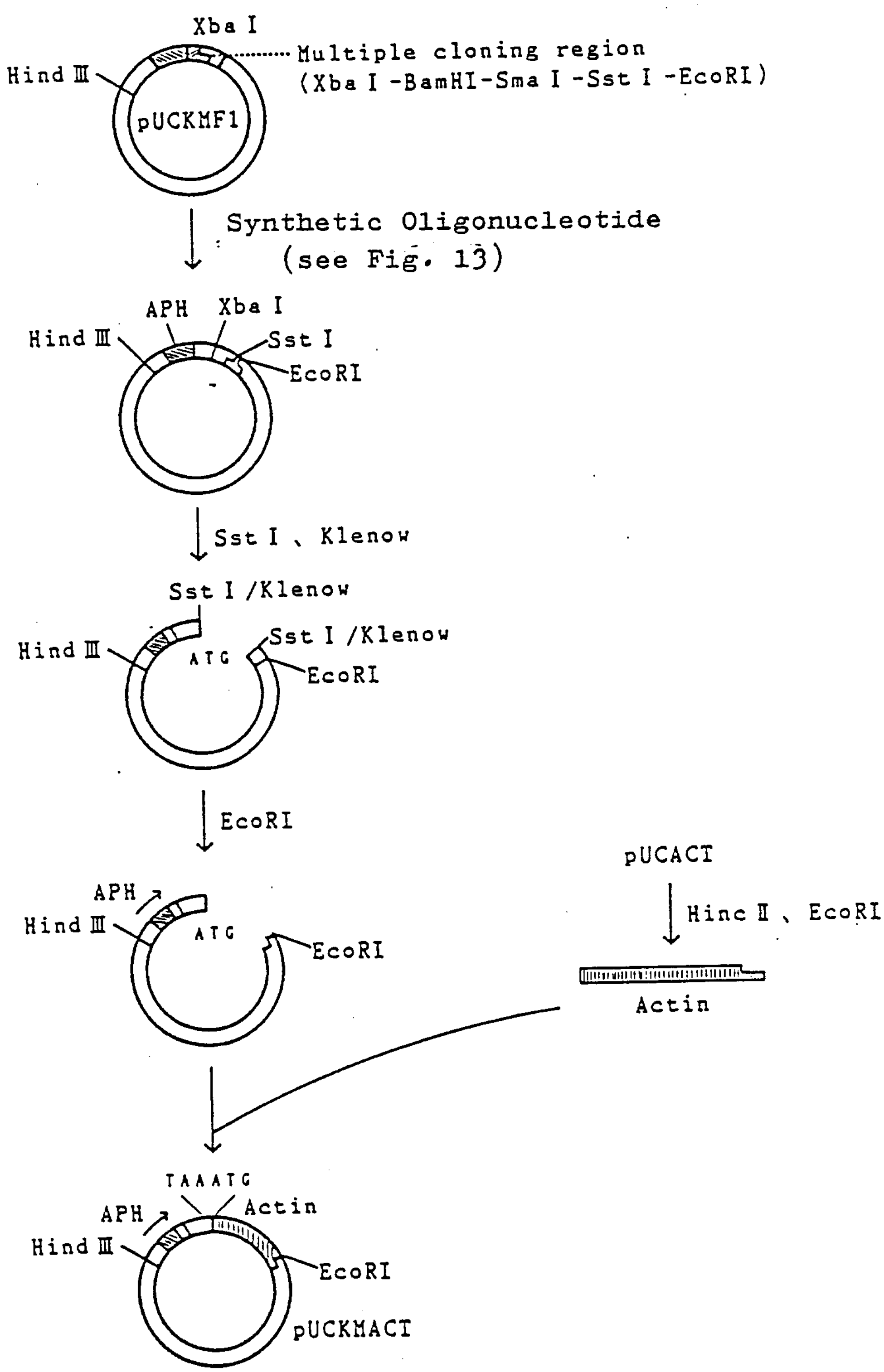
Xba I
Hind III
pUCKMF1
Multiple cloning region
(Xba I -BamHI-Sma I -Sst I -EcoRI)
Synthetic Oligonucleotide
(see Fig. 13)
APH
Xba I
Hind III
Sst I
EcoRI
Sst I 、 Klenow
Sst I /Klenow
Hind III
ATG
Sst I /Klenow
EcoRI
EcoRI
APH
Hind III
ATG
EcoRI
pUCACT
Hinc II 、 EcoRI
Actin
TAAATG
APH
Actin
Hind III
EcoRI
pUCKMACT

Fig. 13

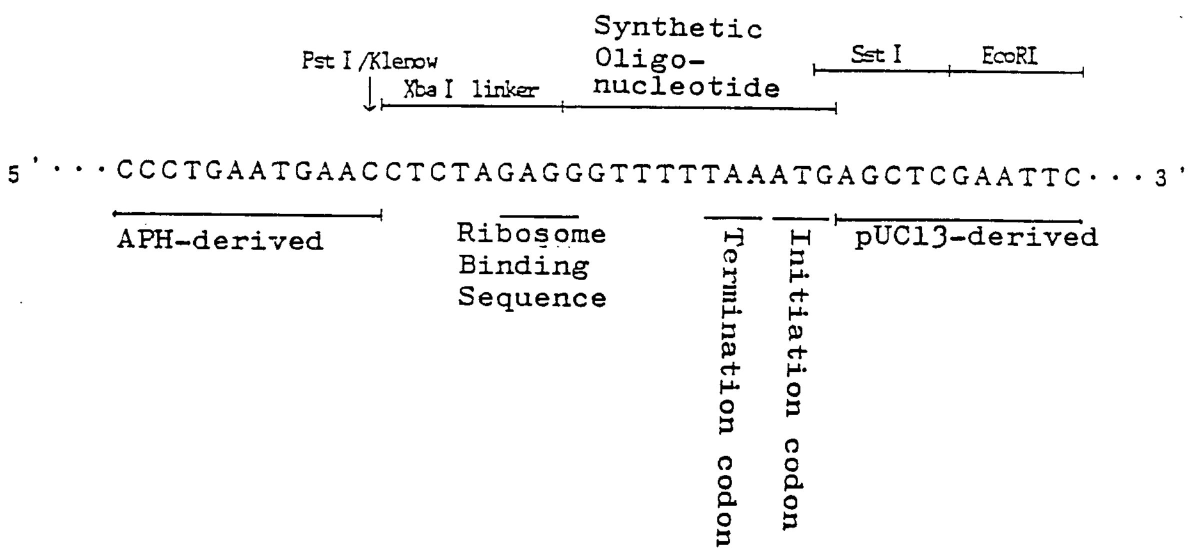

Synthetic Oligo- nucleotide
Pst I /Klenow
Xba I linker
Sst I
EcoRI
5'···CCCTGAATGAACCTCTAGAGGGTTTTTAAATGAGCTCGAATTC···3'
APH-derived
Ribosome Binding Sequence
Termination codon
Initiation codon
pUC13-derived

Fig. 14

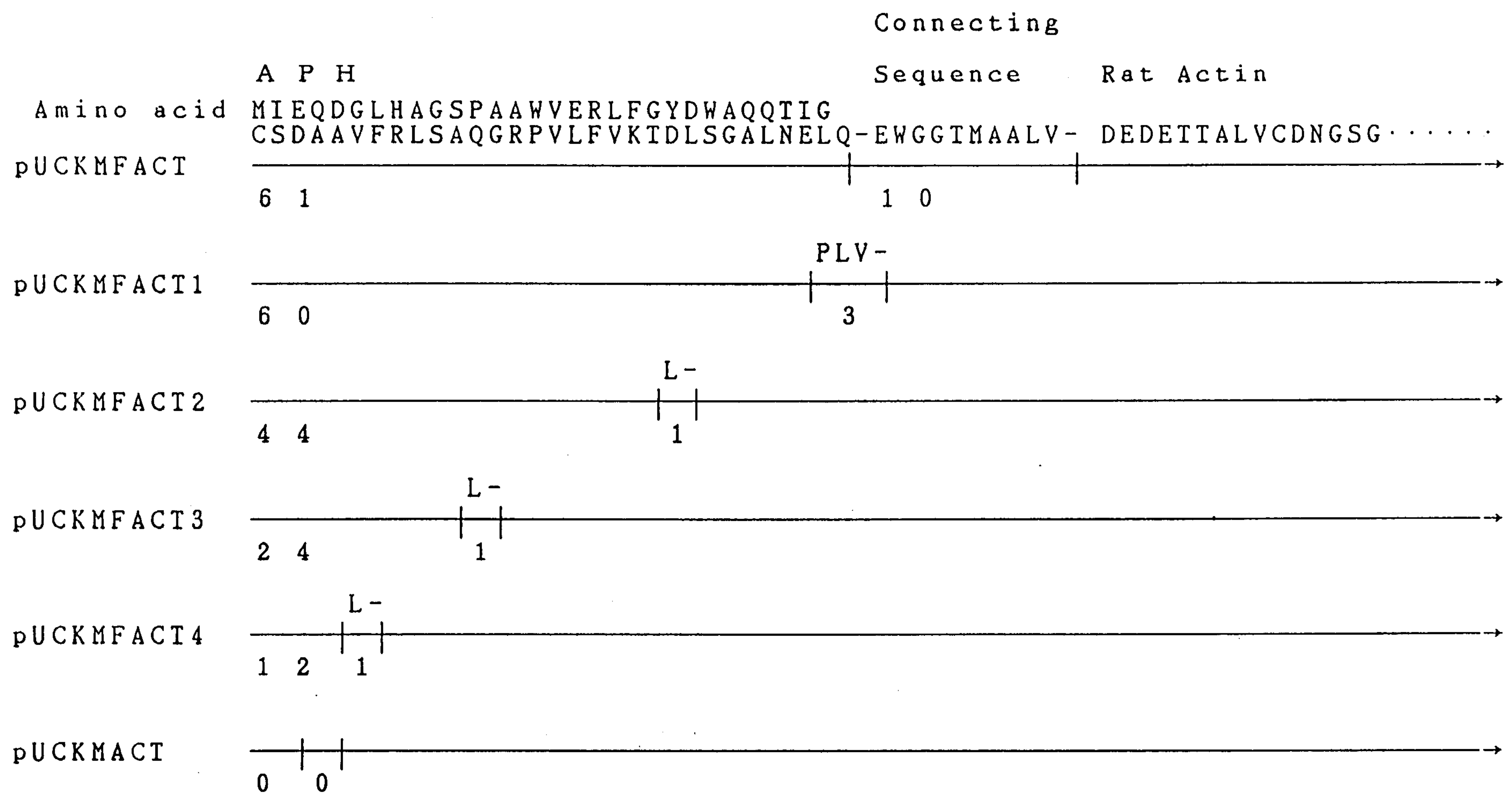
Connecting
Sequence
A P H
Rat Actin
Amino acid
MIEQDGLHAGSPAAWVERLFGYDWAQQTIG
CSDAAVFRLSAQGRPVLFVKTDLSGALNELQ-EWGGTMAALV- DEDETTALVCDNGSG······
pUCKMFACT
6 1
1 0
pUCKMFACT1
PLV-
6 0
3
pUCKMFACT2
L-
4 4
1
pUCKMFACT3
L-
2 4
1
pUCKMFACT4
L-
1 2
1
pUCKMACT
0
0

Fig. 15

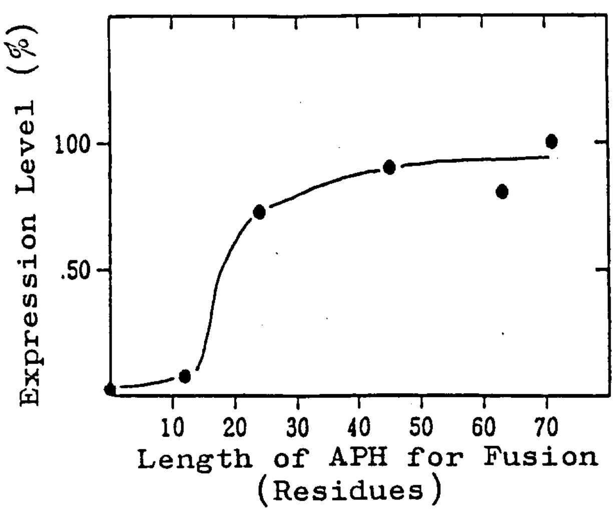
Expression Level (%)
100
.50
10 20 30 40 50 60 70
Length of APH for Fusion
(Residues)